# EUROPEAN PATENT SPECIFICATION

(11) **EP 3 920 786 B1**
(45) Date of publication and mention of the grant of the patent: **11.11.2026**
(21) Application number: 20751870.5
(22) Date of filing: 05.02.2020
(51) Int. Cl.: A61B 5/02

(54) **A SYSTEM, METHOD AND COMPUTER READABLE STORAGE MEDIUM FOR MEDICAL DIAGNOSIS SUPPORT**
SYSTEM, VERFAHREN UND COMPUTERLESBARES SPEICHERMEDIUM ZUR UNTERSTÜTZUNG EINER MEDIZINISCHEN DIAGNOSE
SYSTÈME, PROCÉDÉ ET SUPPORT DE STOCKAGE LISIBLE PAR ORDINATEUR D'AIDE AU DIAGNOSTIC MÉDICAL

(30) Priority: 10.02.2019 US 201962803505 P
(43) Date of publication of application: 15.12.2021
(73) Proprietor: Tyto Care Ltd., Netanya, 4250803 (IL)
(72) Inventor: BYCHKOV, Eyal, 4521464 Hod Hasharon (IL)
(74) Representative: Jaeger, Michael David
(86) International application number: PCT/IL2020/050137
(87) International publication number: WO 2020/161709

(56) References cited:
- US-A1- 2010 121 156
- US-A1- 2014 316 220
- US-A1- 2015 244 903
- US-A1- 2017 124 276

## Description

### TECHNICAL FIELD

The invention relates to a system, method and computer readable storage medium for medical diagnosis support.

### BACKGROUND

The growing costs and complexities of healthcare around the world have made the use of telemedicine - using telecommunication and information technology to provide clinical health care from a distance - more common. Telemedicine is increasingly being viewed as a solution for the increasing demand for affordable and accessible healthcare.

There are many advantages to in-person visits, during which a patient physically visits a medical practitioner (such as a doctor or a nurse, etc.), over telemedicine solutions. One advantage is that in many cases a local medical practitioner meets a plurality of patients from a certain geographical area. Therefore, the local medical practitioner is commonly familiar, not only with the patient himself, but also with the patient's surrounding circles (family members, neighbors, workfellows, classmates, etc.). This provides the local medical practitioner with valuable insight of illnesses in the patient's surrounding environment, that the telemedicine medical practitioner lacks. Another advantage is that a local medical practitioner is familiar with various environmental conditions in the geographical area in which she meets her patients (e.g. water pollution, air pollution, disease outbreaks, radiation levels, weather information, food poisoning information, known diseases, etc.). This provides the local medical practitioner with valuable insight of potential reasons for medical symptoms of the patient, which can enable her to provide more accurate diagnosis.

One of the challenges of telemedicine is how to create a telemedicine appointment that maintains the flexibilities and advantages of providing health care from a distance, while being as effective as an in-person visit, preserving the valuable insight usually in possession of the local medical practitioners, of illnesses in the patient's surrounding geographical or social circles, or about environmental conditions in the patient's whereabouts. Providing such insights to the telemedicine medical practitioner can complement upon the patient's Electronic Health Record (EHR) and enable the telemedicine medical practitioner to provide better diagnosis of medical conditions of patients.

Furthermore, a local medical practitioner can sometimes be proactive and, based on illnesses in the patient's surrounding geographical or social circles, notify an undiagnosed patient of potential infection with the illness. It is necessary to provide similar notification capabilities to the telemedicine medical practitioner, enabling to notify his distant patients of potential infections.

US 2017/124276 discloses a system for displaying patient data and context data.

There is thus a need in the art for a new medical diagnosis support system and method.

### GENERAL DESCRIPTION

A system, method and computer readable storage medium in accordance with the invention is defined in independent claims 1, 8 and 15, respectively.

In accordance with a first aspect of the presently disclosed subject matter, there is provided a medical record management system comprising a processor, the processor configured to: provide a plurality of medical records, each associated with a corresponding patient, wherein each medical record comprises patient identification information and at least one patient attribute, and wherein one or more of the medical records includes one or more past diagnosis previously provided for the corresponding patient; generate, based on the patient attributes, one or more clusters, each cluster associated with at least two medical records, each having at least one shared patient attribute having a value that meets a common condition; receive a diagnosis support information request, including the identification information of a given patient; identify, using the identification information of the given patient, one or more patient-associated clusters of the clusters each associated with the medical record of the given patient, wherein at least one of the medical records of each patient-associated cluster, other than the medical record of the given patient, includes one or more of the past diagnosis; and send a diagnosis support information reply, including at least one past diagnosis of the patient-associated clusters, other than past diagnosis of the given patient.

In some cases, the diagnosis support information reply includes the corresponding shared patient attributes of the identified patient-associated clusters.

In some cases, the diagnosis support information reply includes only past diagnosis with calculated likelihood of relevance to the given patient exceeding a threshold.

In some cases, the patient attributes include one or more of the following: patient surname; patient address; patient type of work; patient place of work; patient age group; or an identifier of a medical data acquisition device used to acquire medical data.

In some cases, the common condition is that the values are equal.

In some cases, the shared patient attribute is a geographical patient attribute and wherein the condition is based on physical proximity.

In accordance with a second aspect of the presently disclosed subject matter, there is provided a medical diagnosis support system comprising a display and a processor, the processor configured to perform the following, for a plurality of patients: obtain: (a) medical information associated with a given patient of the patients; and (b) diagnosis support information, including at least one past diagnosis provided to a previously diagnosed patient, wherein the previously diagnosed patient and the given patient are part of at least one common cluster, the common cluster created based on at least one shared patient attribute of a medical record of the previously diagnosed patient and a medical record of the given patient having a value that meets a common condition; and display the medical information and the diagnosis support information on the display, thereby enabling a medical diagnosing entity, to provide a diagnosis of a medical condition of the given patient, based on (i) the medical information associated with the given patient and (ii) the diagnosis support information.

In some cases, the processor is further configured to: determine one or more medical examinations to be performed on the given patient, based on the medical information and based on the diagnosis support information; and display the medical examinations on the display, thereby enabling a medical diagnosing entity, to recommend additional checks to the given patient.

In some cases, the processor is further configured to: receive the diagnosis from the medical diagnosing entity; and send the diagnosis to the given patient.

In some cases, the processor is further configured to manipulate a queue of patients based on the diagnosis support information, so that a first patient being associated with a first common cluster having a first past diagnosis of a first disease will be ahead of a second patient being associated with a second common cluster having a second past diagnosis of a second disease pre-defined as having an urgency level lower than another urgency level of the first disease.

In some cases, the past diagnosis meets at least one pre-defined criterion.

In some cases, the pre-defined criterion is that the past diagnosis type is diagnosis of a contagious disease.

In some cases, the diagnosis support information further includes a relevance parameter for each of the past diagnosis, the relevance parameter created based on a diagnosis type of the past diagnosis and a cluster type of the common cluster, the relevance parameter being indicative of a likelihood of a relevance of the past diagnosis to the given patient.

In some cases, the common clusters are generated by a medical record management system.

In some cases, at least part of the medical information associated with the given patient is acquired by a medical data acquisition device.

In some cases, the past diagnosis includes one or more of the following: Avian Influenza; Ebola; Hepatitis; HIV/AIDS; Salmonella; or Tuberculosis.

In some cases, the shared patient attributes include one or more of the following: patient surname; patient address; patient type of work; patient place of work; patient age group; and an identifier of a medical data acquisition device used to acquire medical data.

In some cases, the medical diagnosis support system is located remotely from the given patient.

In some cases, the common condition is that the values are equal.

In some cases, the shared patient attribute is a geographical patient attribute and wherein the common condition is based on physical proximity.

In accordance with a third aspect of the presently disclosed subject matter, there is provided a check plan determination system comprising a processor, configured to: receive (a) patient identification information identifying a given patient and (b) diagnosis support information, including at least one past diagnosis of one or more certain medical conditions, provided to a previously diagnosed patient, wherein the previously diagnosed patient and the given patient are part of at least one common cluster, the cluster created based on at least one shared patient attribute of the previously diagnosed patient and the given patient having a value that meets a common condition; and determine, for the given patient, a check plan, based at least on the diagnosis support information, the check plan defining one or more medical examinations to be performed on the given patient, wherein at least one of the medical examinations is required for diagnosing if the given patient has the medical conditions.

In some cases, the check plan includes at least one medical examination that will not have been included in the check plan based on medical information not including the support information.

In some cases, the examinations are performed by a medical data acquisition device.

In some cases, the medical examinations include one or more of the following: body temperature; blood pressure; blood chemistry; or urine examination.

In some cases, the common condition is that the values are equal.

In some cases, the shared patient attribute is a geographical patient attribute and wherein the common condition is based on physical proximity.

In accordance with a fourth aspect of the presently disclosed subject matter, there is provided a medical notification support system comprising a display and a processor, the processor configured to perform the following: obtain: (a) notification support information, including at least one past diagnosis of one or more certain medical conditions, provided to a previously diagnosed patient, wherein the previously diagnosed patient and one or more undiagnosed patients are part of at least one common cluster, the common cluster created based on at least one shared patient attribute of a medical record of the previously diagnosed patient and a medical record of the undiagnosed patient having a value that meets a common condition, and (b) patient identification information identifying the undiagnosed patients; and display the notification support information and the patient identification information on the display, thereby enabling one or more medical personnel, to notify the undiagnosed patients of potential infection with the medical conditions.

In some cases, at least one of the medical personnel is a medical diagnosing entity that is charged with the treatment of the corresponding undiagnosed patient.

In some cases, the medical diagnosing entity is different from the medical diagnosing entity that is charged with the treatment of the previously diagnosed patient.

In some cases, the obtain occurs periodically.

In some cases, the notification to the undiagnosed patients is sent to at least one medical data acquisition device of the undiagnosed patients.

In some cases, the common condition is that the values are equal.

In some cases, the shared patient attribute is a geographical patient attribute and wherein the common condition is based on physical proximity.

In accordance with a fifth aspect of the presently disclosed subject matter, there is provided a method comprising: providing, a plurality of medical records, each associated with a corresponding patient, wherein each medical record comprises patient identification information and at least one patient attribute, and wherein one or more of the medical records includes one or more past diagnosis previously provided for the corresponding patient; generating, based on the patient attributes, one or more clusters, each cluster associated with at least two medical records, each having at least one shared patient attribute having a value that meets a common condition; receiving, a diagnosis support information request, including the identification information of a given patient; identifying, using the identification information of the given patient, one or more patient-associated clusters of the clusters each associated with the medical record of the given patient, wherein at least one of the medical records of each patient-associated cluster, other than the medical record of the given patient, includes one or more of the past diagnosis; and sending, a diagnosis support information reply, including at least one past diagnosis of the patient-associated clusters, other than past diagnosis of the given patient.

In some cases, the diagnosis support information reply includes the corresponding shared patient attributes of the identified patient-associated clusters.

In some cases, the diagnosis support information reply includes only past diagnosis with calculated likelihood of relevance to the given patient exceeding a threshold.

In some cases, the patient attributes include one or more of the following: patient surname; patient address; patient type of work; patient place of work; patient age group; or an identifier of a medical data acquisition device used to acquire medical data.

In some cases, the common condition is that the values are equal.

In some cases, the shared patient attribute is a geographical patient attribute and wherein the condition is based on physical proximity.

In accordance with a sixth aspect of the presently disclosed subject matter, there is provided a method comprising: obtaining, for a plurality of patients: (a) medical information associated with a patient of the patients; and (b) diagnosis support information, including at least one past diagnosis provided to a previously diagnosed patient, wherein the previously diagnosed patient and the given patient are part of at least one common cluster, the common cluster created based on at least one shared patient attribute of a medical record of the previously diagnosed patient and a medical record of the given patient having a value that meets a common condition; and displaying the medical information and the diagnosis support information on the display, thereby enabling a medical diagnosing entity, to provide a diagnosis of a medical condition of the given patient, based on (i) the medical information associated with the given patient and (ii) the diagnosis support information.

In some cases, the method further comprises: determining, one or more medical examinations to be performed on the given patient, based on the medical information and based on the diagnosis support information; and displaying the medical examinations on the display, thereby enabling a medical diagnosing entity, to recommend additional checks to the given patient.

In some cases, the method further comprises: receiving the diagnosis from the medical diagnosing entity; and sending the diagnosis to the given patient.

In some cases, the method further comprises manipulating a queue of patients based on the diagnosis support information, so that a first patient being associated with a first common cluster having a first past diagnosis of a first disease will be ahead of a second patient being associated with a second common cluster having a second past diagnosis of a second disease pre-defined as having an urgency level lower than another urgency level of the first disease.

In some cases, the past diagnosis meets at least one pre-defined criterion.

In some cases, the pre-defined criterion is that the past diagnosis type is diagnosis of a contagious disease.

In some cases, the diagnosis support information further includes a relevance parameter for each of the past diagnosis, the relevance parameter created based on a diagnosis type of the past diagnosis and a cluster type of the common cluster, the relevance parameter being indicative of a likelihood of a relevance of the past diagnosis to the given patient.

In some cases, the common clusters are generated by a medical record management system.

In some cases, at least part of the medical information associated with the given patient is acquired by a medical data acquisition device.

In some cases, the past diagnosis includes one or more of the following: Avian Influenza; Ebola; Hepatitis; HIV/AIDS; Salmonella; or Tuberculosis.

In some cases, the shared patient attributes include one or more of the following: patient surname; patient address; patient type of work; patient place of work; patient age group; or an identifier of a medical data acquisition device used to acquire medical data.

In some cases, the medical diagnosis support system is located remotely from the given patient.

In some cases, the common condition is that the values are equal.

In some cases, the shared patient attribute is a geographical patient attribute and wherein the common condition is based on physical proximity.

In accordance with a seventh aspect of the presently disclosed subject matter, there is provided a method, comprising: receiving (a) patient identification information identifying a given patient and (b) diagnosis support information, including at least one past diagnosis of one or more certain medical conditions, provided to a previously diagnosed patient, wherein the previously diagnosed patient and the given patient are part of at least one common cluster, the cluster created based on at least one shared patient attribute of the previously diagnosed patient and the given patient having a value that meets a common condition; and determining, for the given patient, a check plan, based at least on the diagnosis support information, the check plan defining one or more medical examinations to be performed on the given patient, wherein at least one of the medical examinations is required for diagnosing if the given patient has the medical conditions.

In some cases, the check plan includes at least one medical examination that will not have been included in the check plan based on medical information not including the support information.

In some cases, the examinations are performed by a medical data acquisition device.

In some cases, the medical examinations include one or more of the following: body temperature; blood pressure; blood chemistry; or urine examination.

In some cases, the common condition is that the values are equal.

In some cases, the shared patient attribute is a geographical patient attribute and wherein the common condition is based on physical proximity.

In accordance with a eighths aspect of the presently disclosed subject matter, there is provided a method, comprising: obtaining: (a) notification support information, including at least one past diagnosis of one or more certain medical conditions, provided to a previously diagnosed patient, wherein the previously diagnosed patient and one or more undiagnosed patients are part of at least one common cluster, the common cluster created based on at least one shared patient attribute of a medical record of the previously diagnosed patient and a medical record of the undiagnosed patient having a value that meets a common condition, and (b) patient identification information identifying the undiagnosed patients; and displaying, on a display, the notification support information and the patient identification information on the display, thereby enabling one or more personnel, to notify the undiagnosed patients of potential infection with the medical conditions.

In some cases, at least one of the medical personnel is a medical diagnosing entity that is charged with the treatment of the corresponding undiagnosed patient.

In some cases, the medical diagnosing entity is different from the medical diagnosing entity that is charged with the treatment of the previously diagnosed patient.

In some cases, the obtain occurs periodically.

In some cases, the notification to the undiagnosed patients is sent to at least one medical data acquisition device of the undiagnosed patients.

In some cases, the common condition is that the values are equal.

In some cases, the shared patient attribute is a geographical patient attribute and wherein the common condition is based on physical proximity.

In accordance with a ninth aspect of the presently disclosed subject matter, there is provided a non-transitory computer readable storage medium having computer readable program code embodied therewith, the computer readable program code, executable by at least one processor of a computer to perform a method comprising: providing, a plurality of medical records, each associated with a corresponding patient, wherein each medical record comprises patient identification information and at least one patient attribute, and wherein one or more of the medical records includes one or more past diagnosis previously provided for the corresponding patient; generating, based on the patient attributes, one or more clusters, each cluster associated with at least two medical records, each having at least one shared patient attribute having a value that meets a common condition; receiving, a diagnosis support information request, including the identification information of a given patient; identifying, using the identification information of the given patient, one or more patient-associated clusters of the clusters each associated with the medical record of the given patient, wherein at least one of the medical records of each patient-associated cluster, other than the medical record of the given patient, includes one or more of the past diagnosis; and sending, a diagnosis support information reply, including at least one past diagnosis of the patient-associated clusters, other than past diagnosis of the given patient.

In accordance with a tenth aspect of the presently disclosed subject matter, there is provided a non-transitory computer readable storage medium having computer readable program code embodied therewith, the computer readable program code, executable by at least one processor of a computer to perform a method comprising: obtaining, for a plurality of patients: (a) medical information associated with a given patient of the patients; and (b) diagnosis support information, including at least one past diagnosis provided to a previously diagnosed patient, wherein the previously diagnosed patient and the given patient are part of at least one common cluster, the common cluster created based on at least one shared patient attribute of a medical record of the previously diagnosed patient and a medical record of the given patient having a value that meets a common condition; and displaying the medical information and the diagnosis support information on the display, thereby enabling a medical diagnosing entity, to provide a diagnosis of a medical condition of the given patient, based on (i) the medical information associated with the given patient and (ii) the diagnosis support information.

In accordance with a eleventh aspect of the presently disclosed subject matter, there is provided a non-transitory computer readable storage medium having computer readable program code embodied therewith, the computer readable program code, executable by at least one processor of a computer to perform a method comprising: receiving (a) patient identification information identifying a given patient and (b) diagnosis support information, including at least one past diagnosis of one or more certain medical conditions, provided to a previously diagnosed patient, wherein the previously diagnosed patient and the given patient are part of at least one common cluster, the cluster created based on at least one shared patient attribute of the previously diagnosed patient and the given patient having a value that meets a common condition; and determining, for the given patient, a check plan, based at least on the diagnosis support information, the check plan defining one or more medical examinations to be performed on the given patient, wherein at least one of the medical examinations is required for diagnosing if the given patient has the medical conditions.

In accordance with a twelfth aspect of the presently disclosed subject matter, there is provided a non-transitory computer readable storage medium having computer readable program code embodied therewith, the computer readable program code, executable by at least one processor of a computer to perform a method comprising: obtaining: (a) notification support information, including at least one past diagnosis of one or more certain medical conditions, provided to a previously diagnosed patient, wherein the previously diagnosed patient and one or more undiagnosed patients are part of at least one common cluster, the common cluster created based on at least one shared patient attribute of a medical record of the previously diagnosed patient and a medical record of the undiagnosed patient having a value that meets a common condition, and (b) patient identification information identifying the undiagnosed patients; and displaying, on a display, the notification support information and the patient identification information on the display, thereby enabling one or more medical personnel, to notify the undiagnosed patients of potential infection with the medical conditions.

In accordance with a thirteenth aspect of the presently disclosed subject matter, there is provided a medical diagnosis support system, the medical diagnosis support system comprising a processing resource configured to: obtain medical data acquired from a body of a patient at a given time using a medical data acquisition device; identify and retrieve residual information associated with at least one of: (i) a first location of the patient at the given time, or (ii) at one or more second locations of the patient at one or more corresponding second times earlier than the given time; and display the medical data and the residual information to a medical practitioner, thereby enabling the medical practitioner to provide a diagnosis of a medical condition of the patient.

The retrieved residual information is identified using a first rule set defining a relevance of the residual information for diagnosis purposes based on at least one of: (a) the first location and the given time and metadata defining a time span of relevance of types of the residual information, (b) the second locations, the corresponding second times and the metadata defining a time span of relevance of types of the residual information, (c) known medical conditions of the patient, or (d) the acquired medical data.

In some cases, the medical data is acquired from the patient body and displayed to the medical practitioner during an on-line session between the patient and the medical practitioner.

In some cases, the medical data is acquired from the patient body and displayed to the medical practitioner at a third time, after the given time, wherein the medical data acquisition device is not in communication with the medical diagnosis support system.

In some cases, the residual information includes one or more of: one or more air pollution indicators; one or more water pollution indicators; information of a disease outbreak; information of a radiation level; weather information; food poisoning information; or known diseases at the first location or at the second locations.

In some cases, the residual information is obtained from on-line sources, wherein at least one of the on-line sources is external to the medical diagnosis support system.

In some cases, the medical data acquisition device includes at least one one medical data acquisition sensor, and wherein the medical data includes at least one measurement obtained by the medical data acquisition sensor.

In accordance with a fourteenth aspect of the presently disclosed subject matter, there is provided a medical diagnosis support system, the medical diagnosis support system comprising: a medical data acquisition device comprising a first processing resource and at least one medical data acquisition sensor; and a medical practitioner workstation comprising a second processing resource and a display; wherein the first processing resource is configured to: acquire medical data from a patient using the medical data acquisition sensor, at a given time; send the medical data and location information indicative of a location of the patient at the given time to the medical practitioner device; and wherein, the second processing resource is configured to: receive the medical data and the location information from the medical data acquisition device; retrieve environmental information indicative of environmental conditions at the location; and display the medical information and the environmental information on the display, thereby enabling a medical practitioner operating the medical practitioner workstation to provide a diagnosis of a medical condition of the patient.

The retrieved residual information is identified using a first rule set defining a relevance of the residual information for diagnosis purposes based on at least one of: (a) the first location and the given time and metadata defining a time span of relevance of types of the residual information, (b) the second locations, the corresponding second times and the metadata defining a time span of relevance of types of the residual information, (c) known medical conditions of the patient, or (d) the acquired medical data.

In some cases, the medical data is acquired from the patient body and displayed to the medical practitioner during an on-line session between the patient and the medical practitioner.

In some cases, the medical data is acquired from the patient body and displayed to the medical practitioner at a third time, after the given time, wherein the medical data acquisition device is not in communication with the medical diagnosis support system.

In some cases, the residual information includes one or more of: one or more air pollution indicators; one or more water pollution indicators; information of a disease outbreak; information of a radiation level; weather information; food poisoning information; or known diseases at the first location or at the second locations.

In some cases, the residual information is obtained from on-line sources, wherein at least one of the on-line sources is external to the medical diagnosis support system.

In some cases, the medical data acquisition device includes at least one one medical data acquisition sensor, and wherein the medical data includes at least one measurement obtained by the medical data acquisition sensor.

In accordance with a fifteenth aspect of the presently disclosed subject matter, there is provided a medical diagnosis support method, the method comprising: obtaining medical data acquired from a body of a patient at a given time using a medical data acquisition device; identifying and retrieving residual information associated with at least one of: (i) a first location of the patient at the given time, or (ii) at one or more second locations of the patient at one or more corresponding second times earlier than the given time; and displaying the medical data and the residual information to a medical practitioner, thereby enabling the medical practitioner to provide a diagnosis of a medical condition of the patient.

The retrieved residual information is identified using a first rule set defining a relevance of the residual information for diagnosis purposes based on at least one of: (a) the first location and the given time and metadata defining a time span of relevance of types of the residual information, (b) the second locations, the corresponding second times and the metadata defining a time span of relevance of types of the residual information, (c) known medical conditions of the patient, or (d) the acquired medical data.

In some cases, the medical data is acquired from the patient body and displayed to the medical practitioner during an on-line session between the patient and the medical practitioner.

In some cases, the medical data is acquired from the patient body and displayed to the medical practitioner at a third time, after the given time, wherein the medical data acquisition device is not in communication with the medical diagnosis support system.

In some cases, the residual information includes one or more of: one or more air pollution indicators; one or more water pollution indicators; information of a disease outbreak; information of a radiation level; weather information; food poisoning information; or known diseases at the first location or at the second locations.

In some cases, the residual information is obtained from on-line sources, wherein at least one of the on-line sources is external to the medical diagnosis support system.

In some cases, the medical data acquisition device includes at least one one medical data acquisition sensor, and wherein the medical data includes at least one measurement obtained by the medical data acquisition sensor.

In accordance with a sixteenth aspect of the presently disclosed subject matter, there is provided a medical diagnosis support method, the method comprising: acquiring, by a medical data acquisition device comprising at least one medical data acquisition sensor, medical data from a patient using the medical data acquisition sensor, at a given time; sending, by the medical data acquisition device, the medical data and location information indicative of a location of the patient at the given time to the medical practitioner device; receiving, by a medical practitioner workstation comprising a display, the medical data and the location information from the medical data acquisition device; retrieving, by the medical practitioner workstation, environmental information indicative of environmental conditions at the location; and displaying the medical information and the environmental information on the display, thereby enabling a medical practitioner operating the medical practitioner workstation to provide a diagnosis of a medical condition of the patient.

The retrieved residual information is identified using a first rule set defining a relevance of the residual information for diagnosis purposes based on at least one of: (a) the first location and the given time and metadata defining a time span of relevance of types of the residual information, (b) the second locations, the corresponding second times and the metadata defining a time span of relevance of types of the residual information, (c) known medical conditions of the patient, or (d) the acquired medical data.

In some cases, the medical data is acquired from the patient body and displayed to the medical practitioner during an on-line session between the patient and the medical practitioner.

In some cases, the medical data is acquired from the patient body and displayed to the medical practitioner at a third time, after the given time, wherein the medical data acquisition device is not in communication with the medical diagnosis support system.

In some cases, the residual information includes one or more of: one or more air pollution indicators; one or more water pollution indicators; information of a disease outbreak; information of a radiation level; weather information; food poisoning information; or known diseases at the first location or at the second locations.

In some cases, the residual information is obtained from on-line sources, wherein at least one of the on-line sources is external to the medical diagnosis support system.

In some cases, the medical data acquisition device includes at least one one medical data acquisition sensor, and wherein the medical data includes at least one measurement obtained by the medical data acquisition sensor.

In accordance with a seventeenth aspect of the presently disclosed subject matter, there is provided a non-transitory computer readable storage medium having computer readable program code embodied therewith, the computer readable program code, executable by at least one processor of a computer to perform a method comprising: obtaining medical data acquired from a body of a patient at a given time using a medical data acquisition device; identifying and retrieving residual information associated with at least one of: (i) a first location of the patient at the given time, or (ii) at one or more second locations of the patient at one or more corresponding second times earlier than the given time; and displaying the medical data and the residual information to a medical practitioner, thereby enabling the medical practitioner to provide a diagnosis of a medical condition of the patient.

### BRIEF DESCRIPTION OF THE DRAWINGS

In order to understand the presently disclosed subject matter and to see how it may be carried out in practice, the subject matter will now be described, by way of non-limiting examples only, with reference to the accompanying drawings, in which:
**Fig. 1** is a block diagram schematically illustrating one example of a system for performing a medical examination of a patient by a remote medical practitioner, in accordance with the presently disclosed subject matter;
**Fig. 2** is a schematic illustration of an environment of a system for medical diagnosis support, in accordance with the presently disclosed subject matter;
**Fig. 3** is a block diagram schematically illustrating one example of a medical diagnosis support system, a patient workstation, a medical data acquisition device and a medical practitioner workstation, and various connections therebetween, in accordance with the presently disclosed subject matter;
**Fig. 4** is a block diagram schematically illustrating one example of a medical record management system and a medical diagnosis support system, and various connections therebetween, in accordance with the presently disclosed subject matter;
**Fig. 5** is a block diagram schematically illustrating one example of a check plan determination system, a medical data acquisition device and a medical diagnosis support system, and various connections therebetween, in accordance with the presently disclosed subject matter;
**Fig. 6** is a block diagram schematically illustrating one example of a medical notification support system and a medical data acquisition device, and various connections therebetween, in accordance with the presently disclosed subject matter;
**Fig. 7** is a flowchart illustrating one example of a sequence of operations carried out by a medical record management system for providing cluster based diagnosis support, in accordance with the presently disclosed subject matter;
**Fig. 8** is a flowchart illustrating one example of a sequence of operations carried out by a medical diagnosis support system for providing cluster based diagnosis support, in accordance with the presently disclosed subject matter;
**Fig. 9** is a flowchart illustrating one example of a sequence of operations carried out by a medical diagnosis support system for manipulate a queue of the diagnosis requesting entities, in accordance with the presently disclosed subject matter;
**Fig. 10** is a flowchart illustrating one example of a sequence of operations carried out by a check plan determination system for providing cluster based diagnosis support, in accordance with the presently disclosed subject matter;
**Fig. 11** is a flowchart illustrating one example of a sequence of operations carried out by a medical notification support system for providing cluster based notification support, in accordance with the presently disclosed subject matter;
**Fig. 12** is a flowchart illustrating one example of a sequence of operations carried out for providing residual information to a medical practitioner, in accordance with the presently disclosed subject matter; and
**Fig. 13** is a flowchart illustrating another example of a sequence of operations carried out for providing residual information to a medical practitioner, in accordance with the presently disclosed subject matter.

### DETAILED DESCRIPTION

In the following detailed description, numerous specific details are set forth in order to provide a thorough understanding of the presently disclosed subject matter. However, it will be understood by those skilled in the art that the presently disclosed subject matter may be practiced without these specific details. In other instances, well-known methods, procedures, and components have not been described in detail so as not to obscure the presently disclosed subject matter.

In the drawings and descriptions set forth, identical reference numerals indicate those components that are common to different embodiments or configurations.

Unless specifically stated otherwise, as apparent from the following discussions, it is appreciated that throughout the specification discussions utilizing terms such as "obtaining", "generating", "receiving", "sending", "providing" or the like, include action and/or processes of a computer that manipulate and/or transform data into other data, said data represented as physical quantities, e.g. such as electronic quantities, and/or said data representing the physical objects. The terms "computer", "processor", and "controller" should be expansively construed to cover any kind of electronic device with data processing capabilities, including, by way of non-limiting example, a personal desktop/laptop computer, a server, a computing system, a communication device, a smartphone, a tablet computer, a smart television, a processor (e.g. digital signal processor (DSP), a microcontroller, a field programmable gate array (FPGA), an application specific integrated circuit (ASIC), etc.), a group of multiple physical machines sharing performance of various tasks, virtual servers co-residing on a single physical machine, any other electronic computing device, and/or any combination thereof.

The operations in accordance with the teachings herein may be performed by a computer specially constructed for the desired purposes or by a genera- purpose computer specially configured for the desired purpose by a computer program stored in a non-transitory computer readable storage medium. The term "non-transitory" is used herein to exclude transitory, propagating signals, but to otherwise include any volatile or nonvolatile computer memory technology suitable to the application.

As used herein, the phrase "for example," "such as", "for instance" and variants thereof describe non-limiting embodiments of the presently disclosed subject matter. Reference in the specification to "one case", "some cases", "other cases" or variants thereof means that a particular feature, structure or characteristic described in connection with the embodiment(s) is included in at least one embodiment of the presently disclosed subject matter. Thus, the appearance of the phrase "one case", "some cases", "other cases" or variants thereof does not necessarily refer to the same embodiment(s).

It is appreciated that, unless specifically stated otherwise, certain features of the presently disclosed subject matter, which are, for clarity, described in the context of separate embodiments, may also be provided in combination in a single embodiment. Conversely, various features of the presently disclosed subject matter, which are, for brevity, described in the context of a single embodiment, may also be provided separately or in any suitable sub-combination.

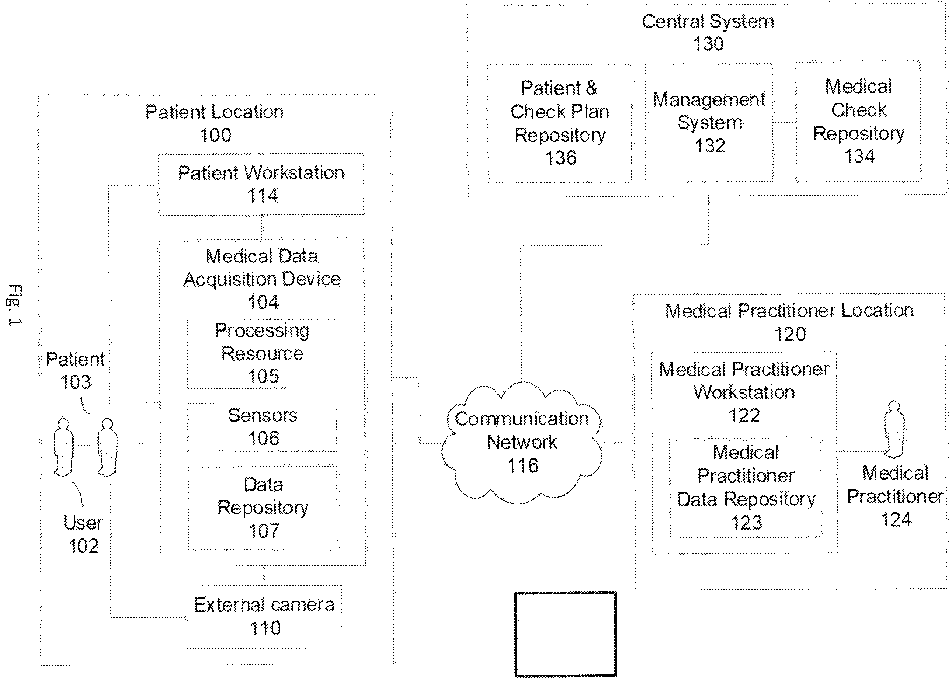

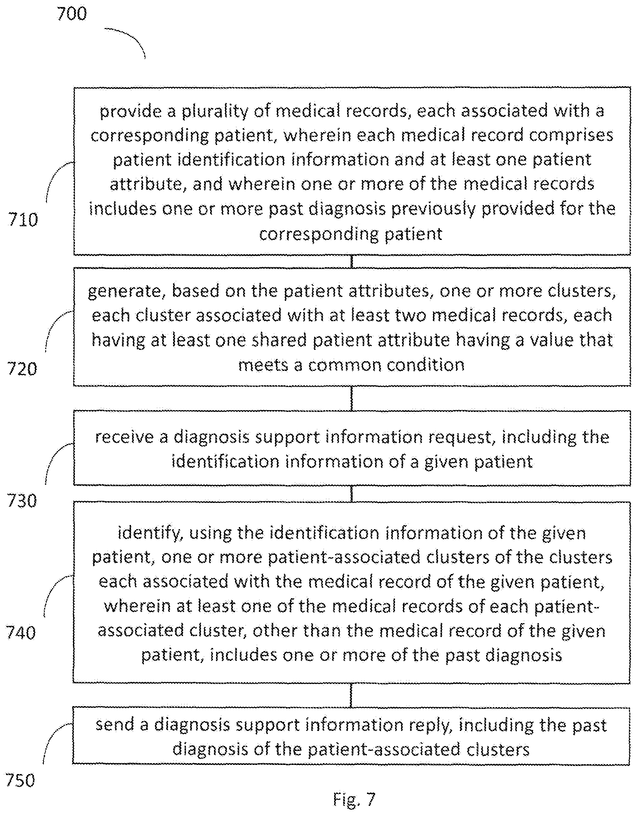

In embodiments of the presently disclosed subject matter, fewer, more and/or different stages than those shown in **Figs. 7-13** may be executed. In embodiments of the presently disclosed subject matter one or more stages illustrated in **Figs. 7-13** may be executed in a different order and/or one or more groups of stages may be executed simultaneously. **Figs. 1-6** illustrate a general schematic of the system architecture in accordance with an embodiment of the presently disclosed subject matter. Each module in **Figs. 1-6** can be made up of any combination of software, hardware and/or firmware that performs the functions as defined and explained herein. The modules in **Figs. 1-6** may be centralized in one location or dispersed over more than one location. In other embodiments of the presently disclosed subject matter, the system may comprise fewer, more, and/or different modules than those shown in **Figs. 1-6****.**

Any reference in the specification to a method should be applied mutatis mutandis to a system capable of executing the method and should be applied mutatis mutandis to a non-transitory computer readable medium that stores instructions that once executed by a computer result in the execution of the method.

Any reference in the specification to a system should be applied mutatis mutandis to a method that may be executed by the system and should be applied mutatis mutandis to a non-transitory computer readable medium that stores instructions that may be executed by the system.

Any reference in the specification to a non-transitory computer readable medium should be applied mutatis mutandis to a system capable of executing the instructions stored in the non-transitory computer readable medium and should be applied mutatis mutandis to method that may be executed by a computer that reads the instructions stored in the non-transitory computer readable medium.

Bearing this in mind, attention is drawn to **Fig. 1****,** a block diagram schematically illustrating one example of a system for performing a medical examination of a patient by a remote medical practitioner, in accordance with the presently disclosed subject matter. A user 102 and a patient 103 (a person or an animal whose medical examination is required) are located at patient location 100, and a medical practitioner 124 is located at a medical practitioner location 120, remote from the patient location 100. In fact, according to the presently disclosed subject matter, the medical practitioner 124 is located at the medical practitioner location 120 that is remote from the patient location 100 so that the medical practitioner 124 does not have direct access to the patient 103 (e.g. it is not located in the same room with the patient 103, nor in any other form of vicinity thereto, so that the medical practitioner 124 cannot himself hold the medical data acquisition device 104 and place it on the body of the patient 103 for acquiring medical data therefrom). In some cases, the medical practitioner 124 can be located in a different room/floor/building/street/city/state/country/continent than the patient 103.

In light of the fact that the medical practitioner 124 is located at a different location than the patient 103, the user 102 is required to operate the medical data acquisition device 104 for acquiring medical data from the patient's 103 body. In this respect, it is to be noted that the user 102 can be the patient 103 whose medical examination is required (in such cases, even though user 102 and patient 103 are shown as separate entities in the drawings, they are in fact the same entity). In other cases, the user 102 can be another person (other than patient 103) that will operate the medical data acquisition device 104 for acquiring medical data from the patient's 103 body, as further detailed herein. In some cases, the user 102 is not a medical practitioner, i.e. the user 102 is not a person specifically trained to acquire medical data from the patient's 103 body, nor is he qualified to diagnose a medical condition of the patient 103 based on medical data acquired from the patient's body.

Attention is drawn to the components within the patient location 100:
The medical data acquisition device 104 comprises (or is otherwise associated with) at least one processing resource 105. Processing resource 105 can be one or more processing units (e.g. central processing units), microprocessors, microcontrollers (e.g. microcontroller units (MCUs)) or any other computing/processing device, which are adapted to independently or cooperatively process data for controlling relevant medical data acquisition device 104 resources and for enabling operations related to medical data acquisition device 104 resources.

Medical data acquisition device 104 further comprises one or more sensors 106 (e.g. camera/s, microphone/s, a thermometer, depth camera/s, an otoscope, a blood pressure sensor, an electrocardiogram (ECG), an ultrasound sensor, an acoustic sensor, a blood saturation sensor, etc.), including at least one sensor capable of acquiring medical data from the patient's 103 body, based on which the medical practitioner 124 can diagnose a medical condition of the patient 103. The medical data can be, for example, body temperature, blood pressure, blood saturation, ECG measurements, audio signals (e.g. of the heart operations or of the lungs), ultrasound signals (e.g. of the heart, of the intestines, etc.), acoustic measurements, body tissue electrical resistance, hardness of body tissues, a heartrate, an image or a video recording of a body organ or a portion of a body organ (whether internal body organ or external body organ), a 3D representation of one or more body organs or portions thereof (whether internal body organ or external body organ), a blood sample analysis, urine samples, throat cultures, saliva samples, or any other parameter associated with one or more physiological characteristic of a patient, based on which diagnosis can be provided.

In some cases, medical data acquisition device 104 can further comprise, or be otherwise associated with, a data repository 107 (e.g. a database, a storage system, a memory including Read Only Memory - ROM, Random Access Memory - RAM, or any other type of memory, etc.) configured to store data, including inter alia patient-related data relating to one or more patients 103 and various medical data acquired from such patients 103 body (e.g. data acquired during a medical examination of the patients using the medical data acquisition device 104), various configuration parameters of the sensor(s) 106, check plans for patient 103 (e.g. defining medical examinations to be performed on patient 103), threshold parameters (e.g. defining required quality levels for various types of measurements), etc. In some cases, data repository 107 can be further configured to enable retrieval and/or update and/or deletion of the stored data. It is to be noted that in some cases, data repository 107 can be distributed across multiple locations, whether within the medical data acquisition device 104 and/or within patient location 100 and/or within central system 130 and/or within medical practitioner location 120 and/or elsewhere. It is to be noted, that in some cases, the relevant information relating to the patient 103 can be loaded into data repository 107 before performing medical examination of the 103 (e.g. upon beginning of a medical examination and/or periodically and/or upon an entity such as the medical practitioner 124 requesting the information).

It is to be noted that in some cases, the medical data acquisition device 104 can be a handheld device, and at least the processing resource 105 and the sensors 106 can be comprised within a housing of the medical data acquisition device 104, that can optionally be a handheld device. In some cases, the sensors can be comprised within removably attachable units configured to be attached to the medical data acquisition device 104. In some cases, the sensors can be external to the medical data acquisition device 104 and in such cases, it may communicate with the medical data acquisition device 104 via a wired connection and/or via a wireless connection (e.g. a WiFi connection).

It is to be further noted that in some cases, medical data acquisition device 104 can further comprise one or more speakers for providing audio recordings to the user 102 (e.g. recordings of a medical practitioner 124 instructing the user 102 how to perform medical examinations, voice instructions generated by the medical data acquisition device 104 instructing the user 102 how to perform medical examinations, etc.). Medical data acquisition device 104 can further comprise a microphone for recording sounds, including voices (e.g. of the user 102 and/or patient 103), in the vicinity of the medical data acquisition device 104, e.g. during medical examinations conducted using the medical data acquisition device 104. Medical data acquisition device 104 can further comprise a display for providing visual output to the user 102 (e.g. a video recording of a remote medical practitioner 124, computer generated instructions instructing the user 102 how to perform medical examinations, indications of quality of an acquired measurement, etc.).

In some cases, medical data acquisition device 104 can communicate, directly, or indirectly, with patient workstation 144 and/or with medical practitioner workstation 122 and/or with central system 130, through communication network 116 (e.g. the Internet), via wired or wireless communication. It is to be noted that such communication can alternatively or additionally be performed utilizing other known communication alternatives, such as a cellular network, Virtual Private Network (VPN), Local Area Network (LAN), etc.

In some cases, a camera 110 can also be located at the patient location 100. Camera 110 (also referred to as "external camera 110") is external to medical data acquisition device 104, in the sense that it is not comprised within the housing of the medical data acquisition device 104. Camera 110 is preferably movable irrespectively of medical data acquisition device 104. Camera 110 is operable to capture visible light, and to generate images or video based on light it captures. Camera 110 may additionally, or alternatively, be sensitive to other parts of the electromagnetic spectrum near the visible spectrum (e.g. to infrared radiation, such as near IR radiation). Camera 110 may be sensitive to the entire visible spectrum (e.g. a commercial-off-the-shelf camera, such as a DSLR camera, a smartphone camera, a webcam camera), or only to a part of it. In some cases, the camera 110 can be a depth camera, capable of generating a 3D representation of the examination process.

Camera 110 is oriented toward the examined patient's 103 body location, in at least some of the time during which medical data acquisition device 104 acquires medical data from the patient's 103 body. Especially, camera 110, when oriented toward the examined patient's 103 body location (as described), is operable to acquire one or more images (that can optionally form a video) which includes at least a part of the patient's 103 body and at least part of the medical data acquisition device 104 when medical data acquisition device 104 (or one or more of the sensors 106) is adjacent to the examined patient 103 body location. Accordingly, images capture by the camera 110 include at least part of the medical data acquisition device 104 and a location on the body of the patient 103 which is currently examined thereby.

In some cases, a patient workstation 114 can also be located at the patient location 100. Patient workstation 114 can be any computer, including a personal computer, a portable computer, a smartphone or any other apparatus with appropriate processing capabilities, including an apparatus which can be, for example, specifically configured for that purpose. The patient workstation 114 can be operated by user 102, for receiving inputs therefrom (e.g. questions to answers, various identification information, etc.), and/or for providing output thereto (showing operational instructions for operating the medical data acquisition device 104, etc.). In some cases, patient workstation 114 can communicate with medical data acquisition device 104 and/or with medical practitioner workstation 122 and/or with central system 130, through communication network 116 (e.g. the Internet), via wired or wireless communication. It is to be noted that such communication can alternatively or additionally be performed utilizing other known communication alternatives, such as a cellular network, Virtual Private Network (VPN), Local Area Network (LAN), etc. It is to be noted that in some cases, patient workstation 114 can comprise the camera 110, and in a more specific example, patient workstation 114 can be a smartphone and camera 110 can be a camera of the smartphone. It is to be noted that in some cases, the processing resources of the patient workstation 114, or of any other computer (located at the patient location 100 or elsewhere), can perform some of the tasks described with reference to processing resource 105 of the medical data acquisition device 104.

Attention is drawn to the components within the medical practitioner location 120:
A medical practitioner workstation 122 is located at the medical practitioner location 120. Medical practitioner workstation 122 can be any computer, including a personal computer, a portable computer, a smartphone or any other apparatus with appropriate processing capabilities, including an apparatus which can be, for example, specifically configured for that purpose. The medical practitioner workstation 122 can receive inputs from the medical practitioner 124 (e.g. instructions and/or questions to be provided to the user 102 and/or patient 103, etc.), and/or provide output to the medical practitioner 124 (showing the medical data acquired by the medical data acquisition device 104, etc.). In some cases, medical practitioner workstation 122 can communicate with medical data acquisition device 104 and/or patient workstation 114 and/or central system 130, through communication network 116 (e.g. the Internet), via wired or wireless communication. It is to be noted that such communication can alternatively or additionally be performed utilizing other known communication alternatives, such as a cellular network, VPN, LAN, etc. In some cases, medical practitioner workstation 122 can communicate with one or more other medical practitioner workstations 122, e.g. when a first medical practitioner operating the medical practitioner workstation 122 is interested in obtaining a second opinion, optionally relating to a certain diagnosis provided by the first medical practitioner, from another medical practitioner.

In some cases, medical practitioner workstation 122 can further comprise, or be otherwise associated with, a medical practitioner data repository 123 (e.g. a database, a storage system, a memory including Read Only Memory - ROM, Random Access Memory - RAM, or any other type of memory, etc.) configured to store data, including inter alia medical data acquired by the medical data acquisition device 104 (optionally including also various metadata relating to such medical data), and other patient-related data relating to one or more patients 103. In some cases, medical practitioner data repository 123 can be further configured to enable retrieval and/or update and/or deletion of the stored data. It is to be noted that in some cases, medical practitioner data repository 123 can be distributed across multiple locations, whether within the medical practitioner location 120 and/or within central system 130 and/or elsewhere. It is to be noted, that in some cases, the relevant information relating to a given examined patient 103 can be loaded into data repository 123 before performing medical examination of a patient 103 (e.g. upon beginning of a medical examination and/or periodically and/or upon an entity such as the medical practitioner 124 requesting the information). In some cases, the medical data can include Electronic Health Records (EHR) data relating to one or more patients 103. In some cases, the EHR data can be obtained through an interface (e.g. over the communication network 116) to a remote EHR system.

In some cases, medical practitioner system 122 can communicate with patient workstation 144 and/or with medical data acquisition device 104 and/or with central system 130, through communication network 116 (e.g. the Internet), via wired or wireless communication. It is to be noted that such communication can alternatively or additionally be performed utilizing other known communication alternatives, such as a cellular network, Virtual Private Network (VPN), Local Area Network (LAN), etc.

In some cases, a central system 130 can exist, for allowing a distributed approach in which medical data and/or other patient-related data can be received by the central system 130 from multiple patient locations 100 and transferred by it to multiple medical practitioner locations 120. Thus, in case the transmitted medical data and/or other patient-related data is received at central system 130, it can be stored in medical check repository 134 and management system 132 can transmit it to a specific medical practitioner location 120 (e.g. via communication network 116 such as the Internet). In some cases, management system 132 can also manage other processes such as, subscribing patients, planning scheduling of patients to available medical practitioners, etc.

It is to be noted that central system 130 is optional to the solution and that central system 130 can be part of the medical practitioner workstation 122. In addition, the communication between the patient workstation 114 and/or the medical data acquisition device 104, and the medical practitioner workstation 122 can be performed directly without the use of or need for a central system 130.

In those cases where a central system 130 exists, it can comprise patient & check plan repository 136 in which various patient-related data, relating to one or more patients 103, is maintained. Such patient-related data can include, for example, patient identification number, patient name, patient age, patient contact details, patient medical record data (such as the patients EHR, information of patient's diseases, sensitivities to medicines, etc.), check plans data (as further detailed below), etc. Central system 130 can further comprise a medical examination repository 134 in which one or more of the following can be stored: (a) medical data acquired by medical data acquisition device 104 (optionally including also various metadata relating to such medical data), (b) user-provided data, provided by the user 102, e.g. using the patient workstation 114, including type-ins and/or voice recording and/or additional info provided by user 102 and relating to the patient 103, and (c) diagnosis data provided by a medical practitioner diagnosing the patient 103. The medical data and/or the user-provided data, can include, for example, voice recordings and/or video recordings and/or values of one or more of the following parameters: body temperature, blood pressure, blood saturation, electrocardiogram (ECG) measurements, audio signals (e.g. of the heart operations or of the lungs), ultrasound signals (e.g. of the heart, of the intestines, etc.), acoustic measurements, body tissue electrical resistance, hardness of body tissues, a heartrate, an image or a video recording of a body organ or a portion of a body organ (whether internal body organ or external body organ), a blood sample analysis, a 3D representation of one or more body organs or portions thereof (whether internal body organ or external body organ), urine samples, throat cultures, saliva samples, or any other parameter associated with one or more physiological characteristic of a patient, based on which diagnosis can be provided. In some cases, one or more of the parameter values can be associated with metadata, such as a timestamp indicative of the time in which the parameter value was acquired, location data indicative of the location at which the parameter value was acquired (e.g. geographical coordinates, WiFi Internet Protocol (IP) address, etc.), a sensor type, information enabling identification of a specific sensor with which the parameter value was acquired, Inertial Navigation System (INS) and/or pressure sensor and/or room humidity and/or room temperature and/or patient orientation and/or room ambient noise level readings acquired during acquisition of the parameter value.

Central system 130 can further comprise management system 132 configured to forward medical data acquired by the medical data acquisition device 104 (whether in a raw form, or any processed version of the raw data acquired by the medical data acquisition device 104) and relating to a patient 103, and optionally other patient-related data relating to the patient 103, to a selected medical practitioner workstation 122 (for example an available medical practitioner workstation 122 or medical practitioner workstation 122 with the shortest queue, e.g. in case where no medical practitioner, out of a plurality of medical practitioners, is currently available). It is to be noted that when providing a central system 130, there may be more than one medical practitioner location 120 and more than one medical practitioner 124 as central system 130 can allow the distributed approach in which data (e.g. medical data and/or other patient-related) can be received by the central system 130 from multiple patient locations 100 and transferred by it to multiple medical practitioner locations 120.

Having described the various components in the patient location 100, in the medical practitioner location 120 and the central system 130, attention is drawn to two exemplary modes of operation of the medical data acquisition device 104: an on-line mode and an off-line mode.

In an on-line mode, a medical examination of the patient 103 is conducted while the medical practitioner 124 is actively involved in the process. In such operation mode, the medical practitioner 124 can be provided with a video or a sequence of images, based on which the medical practitioner 124 provides the user 102 with instructions for positioning the medical data acquisition device 104 with respect to the patient's 103 body. In addition, the medical practitioner 124 can provide the user 102 with instructions for performing a current medical examination (other than positioning instructions) and/or with instructions for performing other medical examinations as part of the medical examination flow. In some cases, the instructions can be audible instructions, acquired by a microphone on the medical practitioner location (e.g. a microphone connected to the medical practitioner workstation 122), and provided to the user 102 via a speaker in the patient location 100 (e.g. a speaker of the medical data acquisition device 104, a speaker of the patient workstation 114, or any other speaker which provides sounds that the user 102 can hear). Additionally, or alternatively to the audible instructions, the instructions can be video instructions provided via a display in the patient location 100 (e.g. a display of the medical data acquisition device 104, a display of the patient workstation 114, or any other display visible to the user 102).

The video that is provided to the medical practitioner 124 can be acquired by a camera comprised within the medical data acquisition device 104 (e.g. one of the sensors 106 can be a camera used for this purpose), and in such case, the medical practitioner 124 can view the part of the patient's body to which the camera is aimed. In additional, or alternative cases, the video can be acquired by an external camera 110 external to the medical data acquisition device 104, and in such cases, the medical practitioner 124 can view the patient 103 and the medical data acquisition device 104 in the same frame. In any case, based on the camera's view, the medical practitioner 124 can provide the user 102 with maneuvering instructions for navigating the medical data acquisition device 104 to a desired spatial disposition with respect to the patient's 103 body. In some cases, the video can be accompanied by a sound recording acquired using a microphone located at the patient location 100 (e.g. a microphone of the medical data acquisition device 104, a microphone of the patient workstation 114, or any other microphone that can acquire a sound recording of sounds at the patient location 100)

Upon arrival of the medical data acquisition device 104 to the desired spatial disposition (from which the medical data can be acquired) with respect to the patient's 103 body, the medical practitioner 124 can instruct the user 102 to acquire the medical data, or it can operate the sensors 106 himself to acquire the medical data. In some cases, the medical practitioner 124 can also remotely control various parameters of the sensors 106, e.g. through medical practitioner workstation 122.

It is noted that medical data acquisition device 104 can be located outside the body of the patient when acquiring the medical data. Nevertheless, in some cases some parts of medical data acquisition device 104 may enter the body of the patient (e.g. a needle penetrating the skin and/or a blood vessel, a sensor entering a body orifice such as the ear or the mouth, and so on). Even in such cases, the greater part of medical data acquisition device 104 can be located outside the body at the time of measurement.

The medical data acquired by the medical data acquisition device 104 can be transmitted to the medical practitioner workstation 122 (directly, or through the patient workstation 114 and/or through the central system 130 where it can be stored in the medical check repository 134 in association with the patient 103 from which the medical data was acquired), where it can be stored in medical practitioner data repository 123 in association with the patient 103 from which the medical data was acquired.

The medical practitioner 124 (e.g. a doctor, a nurse, a medic, etc., including any other person with the know-how and skill to acquire and/or analyze medical data), located at medical practitioner location 120, can review the acquired medical data, for example using medical practitioner workstation 122. It is to be noted that patient workstation 114, medical practitioner workstation 122 and central system 130 can include a display (e.g. LCD screen), and a keyboard or any other suitable input/output devices.

In some cases, medical practitioner 124 can provide feedback data (e.g. by transmitting corresponding instructions to patient workstation 114 and/or to medical data acquisition device 104) to user 102, such as a diagnosis, one or more prescriptions, or instructions to perform one or more additional medical examinations. Alternatively, or additionally, medical practitioner 124 can transmit feedback data to central system 130, which, in turn, can optionally transmit the feedback data to patient workstation 114 and/or to the medical data acquisition device 104 (e.g. via the communication network 116).

In some cases, the medical data acquisition device 104 and/or the patient workstation 114 can be configured to provide the user 102 with an indication of a quality of a signal acquired by the sensors. In such cases, the medical data acquisition device 104 and/or the patient workstation 114 can be configured to determine the signal quality and display an appropriate indication on a display visible by the user 102 (e.g. a display of the medical data acquisition device 104 and/or a display of the patient workstation 114). In some cases, upon the signal quality not meeting pre-defined thresholds, the medical data acquisition device 104 and/or the patient workstation 114 can be configured to provide the user 102 with instructions for improving the acquired signal quality (e.g. instructions to reposition the medical data acquisition device 104, instructions to reduce ambient noise, etc.).

In an off-line mode, a medical examination of the patient 103 is conducted while no medical practitioner 124 is actively involved in the process. In such operation mode, the medical data acquisition device 104 can provide the user 102 with audio and/or video navigation instructions for navigating the medical data acquisition device 104 to a desired spatial disposition with respect to the patient's 103 body. The navigation instructions can be determined by the medical data acquisition device 104 and/or by the patient workstation 114 using information obtained from an Inertial Navigation System (INS), that can optionally be part of the sensors 106, and/or using matching of reference points within reference images and images acquired by a camera comprised within the medical data acquisition device 104 and/or by external camera 110. The navigation instructions can be provided via a speaker and/or a display of the medical data acquisition device 104 and/or of the patient workstation 114 and/or of any other device located near the user 102 in a manner that enables a user to hear and/or see the navigation instructions.

Upon arrival of the medical data acquisition device 104 to the desired spatial disposition (from which the medical data can be acquired) with respect to the patient's 103 body, the user 102 can operate the medical data acquisition device 104 to acquire medical data, or alternatively, the medical data acquisition device 104 can acquire the medical data automatically.

In some cases, the medical data acquisition device 104 and/or the patient workstation 114 can be configured to provide the user 102 with an indication of a quality of a signal acquired by the sensors. In such cases, the medical data acquisition device 104 and/or the patient workstation 114 can be configured to determine the signal quality and display an appropriate indication on a display visible by the user 102 (e.g. a display of the medical data acquisition device 104 and/or a display of the patient workstation 114). In some cases, upon the signal quality not meeting pre-defined thresholds, the medical data acquisition device 104 and/or the patient workstation 114 can be configured to provide the user 102 with instructions for improving the acquired signal quality (e.g. instructions to reposition the medical data acquisition device 104, instructions to reduce ambient noise, etc.).

It is noted that medical data acquisition device 104 can be located outside the body of the patient when acquiring the medical data. Nevertheless, in some cases some parts of medical data acquisition device 104 may enter the body of the patient (e.g. a needle penetrating the skin and/or a blood vessel, a sensor entering a body orifice such as the ear or the mouth, and so on). Even in such cases, the greater part of medical data acquisition device 104 can be located outside the body at the time of measurement.

The medical data acquired by the medical data acquisition device 104 can be transmitted to a medical practitioner workstation 122 (directly, or through the patient workstation 114 and/or through the central system 130 where it can be stored in the medical check repository 134 in association with the patient 130 from which the medical data was acquired), where it can be stored in medical practitioner data repository 123 in association with the patient 130 from which the medical data was acquired.

The medical practitioner 124 (e.g. a doctor, a medic, etc., including any other entity (human or computerized) with the know-how and skill to acquire and/or analyze medical data), located at medical practitioner location 120, can review the acquired medical data, for example using a display and/or a speaker and/or any other suitable output device of the medical practitioner workstation 122. It is to be noted that patient workstation 114, medical practitioner workstation 122 and central system 130 can include a display (e.g. LCD screen), and a keyboard or any other suitable input/output devices.

In some cases, medical practitioner 124 can provide feedback data (e.g. by transmitting corresponding instructions to patient workstation 114 and/or to medical data acquisition device 104) to user 102, such as a diagnosis, one or more prescriptions, or instructions to perform one or more additional medical examinations. Alternatively, or additionally, medical practitioner 124 can transmit feedback data to central system 130, which, in turn, can optionally transmit the feedback data to patient workstation 114 (e.g. via the communication network 116). As indicated herein, the feedback data can be provided to the user 102 via an output device (e.g. a display, a speaker, etc.) of the medical data acquisition device 104 and/or of the patient workstation, or of any other device that is capable of providing the respective output to the user 102.

It is to be noted that in some cases, the medical practitioner data repository 123 and/or the data repository 107, and or the medical check repository 134 and/or the patient & check plan repository 136 can be the same single data repository, whether distributed or not, that is accessible by all relevant entities.

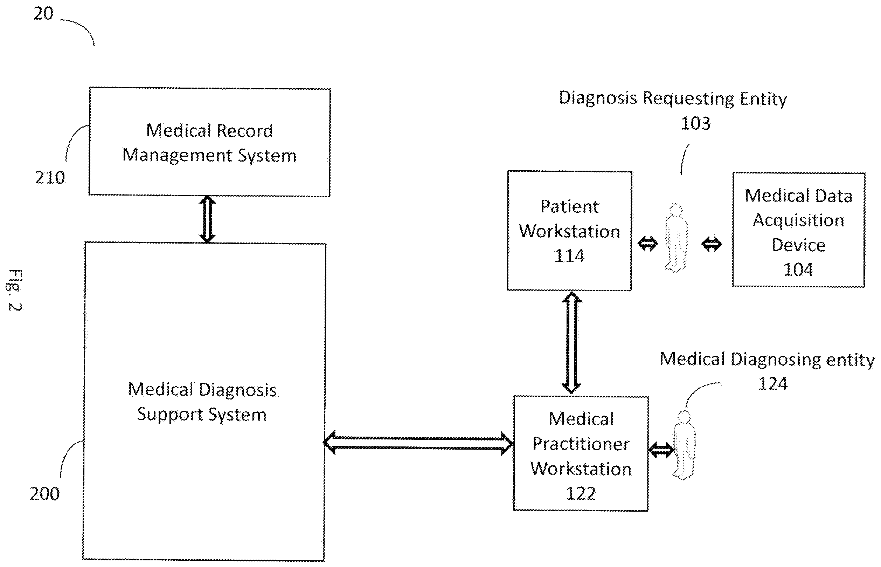

Turning to **Fig. 2****,** there is show a schematic illustration of an environment of a system for medical diagnosis support, in accordance with the presently disclosed subject matter.

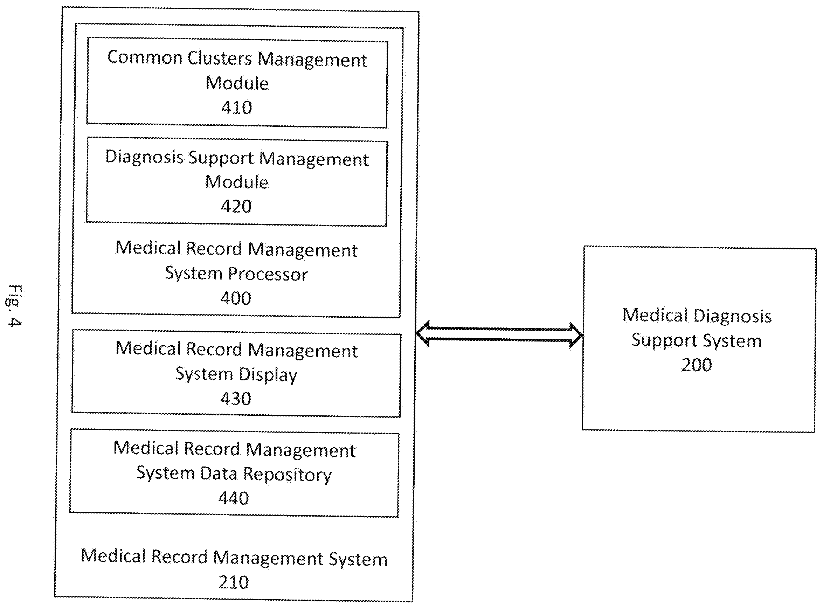

According to certain examples of the presently disclosed subject matter, the environment 20, includes one or more medical record management systems 210, each having a processor, a data repository and optionally also a display (e.g. LCD screen) and/or a keyboard or any other suitable input/output devices, as further detailed herein, inter alia with reference to Fig. 4.

The medical record management system 210 holds, in a data repository, a plurality of medical records, each medical record is associated with a corresponding patient 103. Each medical record comprises patient identification information (e.g. a patient identification number, patient's biometric identifiers, such as fingerprints, DNA, iris recognition, etc.) which uniquely identifies the corresponding patient 103. In addition, each medical record comprises patient attributes (e.g. patient name, age, age group, address, type of work, place of work, location information, sensitivities to medicines, an identifier of a medical data acquisition device 104 used to acquire medical data, etc.) of the corresponding patient 103. Such attributes' values can be used in order to cluster the medical records into groups as further detailed herein.

In addition, at least part of the medical records further includes one or more past diagnosis, previously provided to the corresponding patient 103, e.g. by a medical practitioner 124 of the corresponding patient 103 during a past real (i.e. in-person) or virtual patient visit. For example, a medical record for a specific patient 103, may include a Social Security Number (SSN) uniquely identifying patient 103, patient attributes, such as name, address, workplace address, location information (e.g. obtained from a GPS of a smartphone of the patient 103), with the corresponding values and one or more past diagnoses given to patient 103 during his past real/virtual patient visits to a medical practitioner 124 - e.g. one of the past diagnosis given at a recent patient visit diagnosing the patient 103 as suffering from a flu or asthma or any other medical condition (whether temporary or permanent).

The medical record management system 210 can be further configured to cluster the medical records into groups, based on the patient attributes. Medical records who share attributes' values will be grouped in the same cluster. When the value of attributes of two or more medical records meets a common condition (e.g. equality of the values, physical proximity of the values, values that are part of a predefined group, etc.) the attribute's value is determined by the medical record management system 210 to be a shared attributes' value.

For example, based on an identical value for the place of work patient attribute, a workplace "X" cluster may be created, holding the medical records of people working at the same workplace "X". In another example, the medical record management system 210 may create a neighborhood "Y" cluster, holding the medical records that have an address value that is within a specific geographical area defining the geographic boundaries of neighborhood "Y". Another example may be based on a physical proximity condition, wherein the medical record management system 210 may create a contiguity "Z" cluster, holding the medical records of all patients 103 having been in physical proximity of each other (e.g. in a location less than 5 meters of each other) during a given time frame (e.g. in the last three days).

In some cases, the medical record management system 210 can be located at the medical practitioner location 120. In such cases, the medical record management system 210 may be incorporated into the medical practitioner workstation 122 or may operate as a stand-alone system communicated with via a local network at the medical practitioner location 120. In other cases, the medical record management system 210 can be part of central system 130, or it can be a separate system located at the central system 130 location or at other locations. In such cases, the medical record management system 210 may communicate with a medical diagnosis support system 200 and/or with a patient workstation 114 and/or with a medical practitioner workstation 122 via a communication network 116.

Environment 20 can further include one or more diagnosis requesting entities 103 (it is to be noted that the terms patient and diagnosis requesting entity are used herein interchangeably), whose medical diagnosis is required to be made by one or more medical diagnosing entities 124 (it is to be noted that the terms medical practitioner and medical diagnosing entity are used herein interchangeably). The diagnosis requesting entity 103 can be located at patient location 100 which can optionally be located remotely from a medical practitioner location 120 (e.g. in a different room / floor / building / street / city / state / country / continent than the diagnosis requesting entity 103).

Each diagnosis requesting entity 103 may request a medical diagnosis from one or more medical diagnosing entities 124 (whether randomly selected by the central system 130 or specifically identified by the diagnosis requesting entity 103). The request for medical diagnosis may be made in person, e.g. as part of a real in-person patient visit of the diagnosis requesting entity 103 at the premises of the medical diagnosing entity 124 (i.e. at the medical practitioner location 120), or the request for medical diagnosis may be made by the diagnosis requesting entity 103 from the patient workstation 114 as part of a virtual patient visit. In some cases, the request for diagnosis can be inputted by the medical diagnosing entity 124 using the medical practitioner workstation 122, e.g. when a first medical diagnosing entity 124 operating the medical practitioner workstation 122 is interested in obtaining a second opinion, optionally relating to a certain diagnosis provided by the first medical diagnosing entity 124, from a second medical diagnosing entity 124.

As part of the diagnosis request, the medical diagnosing entity 124 may have access to medical data that is provided by the diagnosis requesting entity 103 (e.g. indications of certain physiological phenomena such as headaches, stomach aches, nausea, diarrhea, etc.) and/or acquired from the diagnosis requesting entity's 103 body, optionally by a medical data acquisition device 104. The medical data may include attribute values representing a physiological characteristic of a diagnosis requesting patient 103 (e.g. body temperature, blood pressure, ECG measurements, etc.) and in some cases, one or more of the attribute values can be associated with metadata, such as a timestamp indicative of the time in which the value was acquired, and/or an ID number identifying the medical data acquisition device 104 that was used to acquire that value, etc.

For example, a specific diagnosis requesting entity 103, may utilize his patient workstation 114 to initiate a virtual patient visit to a medical diagnosing entity 124. The diagnosis requesting entity 103 will enter a patient identification information, i.e. his SSN, his own body temperature, e.g. as acquired by patient 103 using a medical data acquisition device 104, complaints of the patient relating to medical phenomena (such as headaches) and request a diagnosis from the medical diagnosing entity 124. The medical diagnosing entity 124 may access the request for diagnosis, with all accompanying medical data, on the medical practitioner workstation 122.

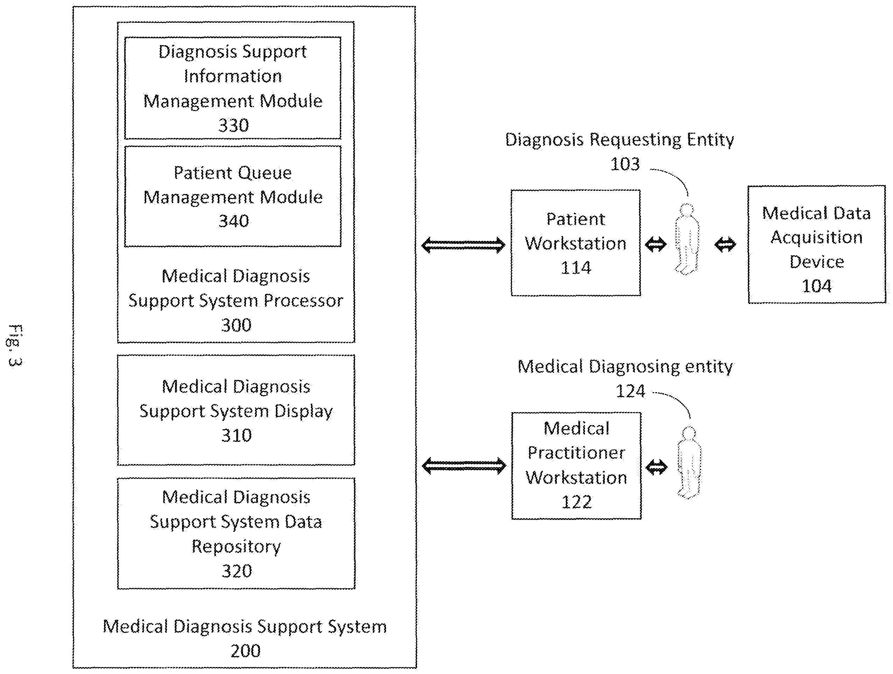

Environment 20, may further include one or more medical diagnosis support systems 200, each having a processor, a data repository and optionally also a display (e.g. LCD screen), and/or a keyboard or any other suitable input/output devices, as further detailed herein, inter alia with reference to Fig. 3. In some cases, the medical diagnosis support system 200 can be located at the medical practitioner location 120. In such cases, the medical diagnosis support system 200 may be incorporated into the medical practitioner workstation 122 or may operate as a stand-alone system communicated with via a local network at the medical practitioner location 120. In other cases, the medical diagnosis support system 200 can be part of central system 130 or it can be a standalone system located at the location of the central system 130 or at any other location. In such cases, the medical diagnosis support system 200 may communicate with a medical record management system 210 and/or with a patient workstation 114 and/or with a medical practitioner workstation 122 via a communication network 116.

The medical diagnosing entity 124 may utilize the medical diagnosis support system 200 to obtain diagnosis support information for a given diagnosis requesting entity 103.

In some cases, the diagnosis support information includes at least one past diagnosis provided to a previously diagnosed patient, wherein the previously diagnosed patient and the given diagnosis requesting entity 103 are part of at least one common cluster. Optionally, the diagnosis support information may include only past diagnosis with calculated likelihood of relevance to the given diagnosis requesting entity 103 that exceeds a certain threshold, e.g. only past diagnosis given in recent timeframe will be included in the diagnosis support information. In addition, the diagnosis support information may include the shared attributes' values, which were the cause for clustering the common clusters associated with diagnosis requesting entity 103. The diagnosis support information may be optionally obtained from a medical record management system 210, as detailed herein, inter alia Fig. 4.

The diagnosis support information includes residual information that is indicative of environmental conditions (e.g. water pollution, air pollution, disease outbreaks, radiation levels, weather information, food poisoning information, known diseases, etc.) in the location of the given diagnosis requesting entity 103 at the point in time in which the given diagnosis requesting entity 103 requests diagnosis, or in past locations of the given diagnosis requesting entity 103 (e.g. obtained from a location monitoring device such as a GPS receiver, or from any other source).

The diagnosis support information may be displayed on the medical practitioner workstation 122, thereby enabling the medical diagnosing entity 124, to provide a diagnosis of a medical condition of the given diagnosis requesting entity 103.

With reference to the clustering, continuing the first example above, a given diagnosis requesting entity 103, that is part of a specific workplace "X" cluster, may request a diagnosis from a medical diagnosing entity 124. The medical diagnosing entity 124 may utilize a medical diagnosis support system 200 to obtain and display diagnosis support information for the given diagnosis requesting entity 103, including past diagnoses given to patients that are part of the workplace "X" cluster that meet a certain relevance threshold, e.g. a diagnosis for a certain food related condition given the day before to a co-worker of diagnosis requesting entity 103, thereby enabling the medical diagnosing entity 124, to provide a diagnosis of a medical condition of the given diagnosis requesting entity 103, taking into consideration the possibility of exposure of diagnosis requesting entity 103 to the same food related condition.

Continuing the second example above, a given diagnosis requesting entity 103, that is part of a specific neighborhood "Y" cluster, may request a diagnosis from a medical diagnosing entity 124. The medical diagnosing entity 124 may utilize a medical diagnosis support system 200 to obtain and display diagnosis support information for the given diagnosis requesting entity 103, including past diagnoses given to patients that are part of the neighborhood "Y" cluster that meet a certain relevance threshold, e.g. a diagnosis for a contiguous disease given two days ago to a neighbor of diagnosis requesting entity 103, thereby enabling the medical diagnosing entity 124, to provide a diagnosis of a medical condition of the given diagnosis requesting entity 103, taking into consideration the possibility of exposure to the contiguous disease.

Continuing the third example above, a given diagnosis requesting entity 103, that is part of a specific contiguity "Z" cluster, may request a diagnosis from a medical diagnosing entity 124. The medical diagnosing entity 124 may utilize a medical diagnosis support system 200 to obtain and display diagnosis support information for the given diagnosis requesting entity 103, including past diagnoses given to patients that are part of the contiguity "Z" cluster that meet a certain relevance threshold, e.g. a diagnosis for a contiguous disease given today to a person that was in physical proximity of 5 meters of diagnosis requesting entity 103 sometime in the last three days, thereby enabling the medical diagnosing entity 124, to provide a diagnosis of a medical condition of the given diagnosis requesting entity 103, taking into consideration the possibility of exposure to the contiguous disease.

As another example, in which the diagnosis support information includes residual information that is indicative of environmental conditions, a given diagnosis requesting entity 103 that is known to be asthmatic, seeks diagnosis as she suffers from breathing difficulties. It may be the case that the given diagnosis requesting entity 103 is located at a geographical area that is known to have high air pollution levels at the time the given diagnosis requesting entity 103 seeks diagnosis. A medical diagnosing entity 124 that is unfamiliar with the air pollution in the given diagnosis requesting entity 103 location, may be unable to identify that the reason that the given diagnosis requesting entity 103 has breathing difficulties is the high air pollution level in its location, and therefore he may provide erroneous diagnosis. Having diagnosis support information that includes residual information indicative of environmental conditions at the given diagnosis requesting entity 103 location can enable the medical diagnosing entity 124 to provide an accurate diagnosis.

The medical diagnosis support system 200 may further determine one or more additional medical examinations to be performed in order to obtain additional medical data from a certain diagnosis requesting entity 103. The additional medical examinations are determined based on the medical information and based on the diagnosis support information. For example, based on the diagnosis support information, which includes a relevant past diagnosis of flu in the cluster of a given diagnosis requesting entity 103, the medical diagnosis support system 200 may display to the medical diagnosing entity 124 a suggested body temperature additional medical exam (assuming that no body temperature was performed on the diagnosis requesting entity 103). As another Example, the additional medical examinations can be determined based on the residual information that is indicative of environmental conditions, so that if the medical diagnosing entity 124 is aware of high air pollution levels at the location of the given diagnosis requesting entity 103, she can instruct the given diagnosis requesting entity 103 to perform a lungs oscillation examination in order to enable the medical diagnosing entity 124 to provide an accurate diagnosis.

The additional medical examinations may be displayed on the display, thereby enabling a medical diagnosing entity 124, to recommend additional medical examinations to be performed on the diagnosis requesting entity 103.

Optionally, the additional medical examinations can be automatically updated onto a medical data acquisition device 104 of the diagnosis requesting entity 103, thereby enabling the diagnosis requesting entity 103 to perform the additional medical examinations utilizing the medical data acquisition device 104, i.e. with no need to manually set up the medical data acquisition device 104 to be suitable for the performance of the additional medical examinations. Alternatively, or additionally, the additional medical examination can be introduced into a check plan (stored on a patient & check plan repository 136) associated with the diagnosis requesting entity 103, so that the next time the medical data acquisition device 104 is used to collect medical data from the diagnosis requesting entity 103, it will also perform the additional medical examination. Continuing the example above, the required temperature exam may be optionally updated automatically onto the medical data acquisition device 104 of the patient 103, thereby enabling patient 103 to check his temperature with medical data acquisition device 104 or it may optionally be added to the check plan associated with the diagnosis requesting entity 103, stored on the patient & check plan repository 136, therefore, the next time the medical data acquisition device 104 is used to collect medical data from the diagnosis requesting entity 103, it will also perform the additional medical examination.

The medical diagnosis support system 200 may further receive a diagnosis of a medical condition of a given diagnosis requesting entity 103, provided by a medical diagnosing entity 124, from the medical diagnosing entity 124, e.g. via a medical practitioner workstation 122, and send the diagnosis to the given diagnosis requesting entity 103, e.g. via a patient workstation 114.

The medical diagnosis support system 200 may further be configured to manipulate a queue of the diagnosis requesting entities 103, e.g. a queue of diagnosis requesting entities 103 awaiting to receive a diagnosis from a medical diagnosing entity 124, either by physically waiting at the medical practitioner location 120 or by sending requests for diagnosis to a medical practitioner workstation 122 that, as indicated herein, can be remote. The manipulation is based on the diagnosis support information. Accordingly, for example, a first diagnosis requesting entity 103 being associated with a first common cluster having a first past diagnosis of a first disease will be ahead of a second diagnosis requesting entity 103 being associated with a second common cluster having a second past diagnosis of a second disease pre-defined as having an urgency level lower than another urgency level of the first disease, even if the second diagnosis requesting entity 103 entered the queue before the first diagnosis requesting entity 103. As another example, a first asthmatic diagnosis requesting entity 103 located at an area with a first air pollution levels will be ahead of a second asthmatic diagnosis requesting entity 103 located at an area with air pollution levels lower than the first air pollution levels.

In a specific example related to the clusters, if a first diagnosis requesting entity 103, being associated with a specific cluster having a past diagnosis of a disease with a low urgency level (e.g. common flu), and a second diagnosis requesting entity 103, being associated with a specific cluster having a past diagnosis of a disease with a high urgency level (e.g. Ebola), are awaiting to receive a diagnosis from a given medical diagnosing entity 124, than the medical diagnosis support system 200 may make sure that the second diagnosis requesting entity 103 is ahead of the first diagnosis requesting entity 103 in the queue to receive diagnosis from the given medical diagnosing entity 124.

In another example related to the clusters, a diagnosis requesting entity 103, being associated with a specific cluster having a relevant past diagnosis of an infectious disease is advanced over a diagnosis requesting entity 103, being associated with a specific cluster having a relevant past diagnosis of a non-infectious disease.

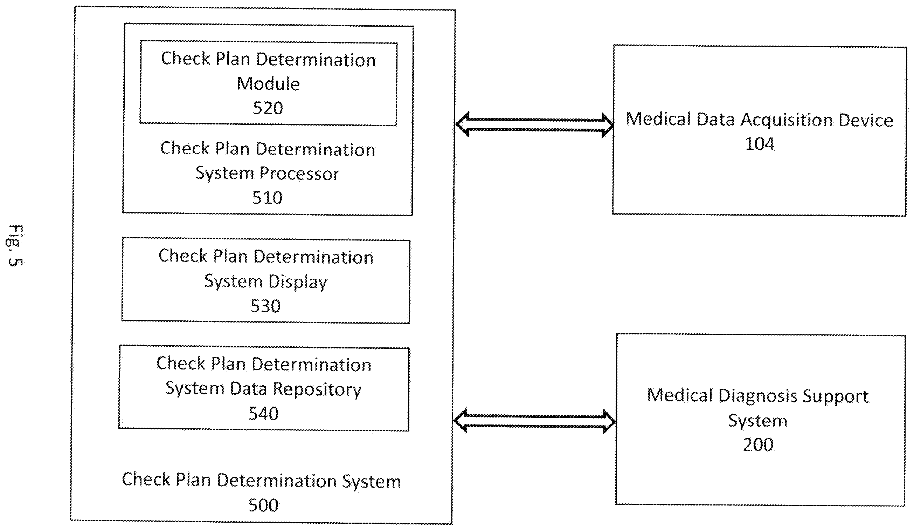

In addition, after obtaining the diagnosis support information for a given diagnosis requesting entity 103, a check plan determination system 500 may determine a check plan, including medical examinations for the conditions included in the past diagnoses, found in the given diagnosis requesting entity's 103 common clusters. In addition, the check plan determination system 500 may determine updated check plans in response to a diagnosis of a certain medical condition given to another patient 103. The updated check plans will be given to all undiagnosed patients 103 included in the common clusters, in order to examine potential infection with the medical condition, as detailed herein, inter alia Fig. 5.

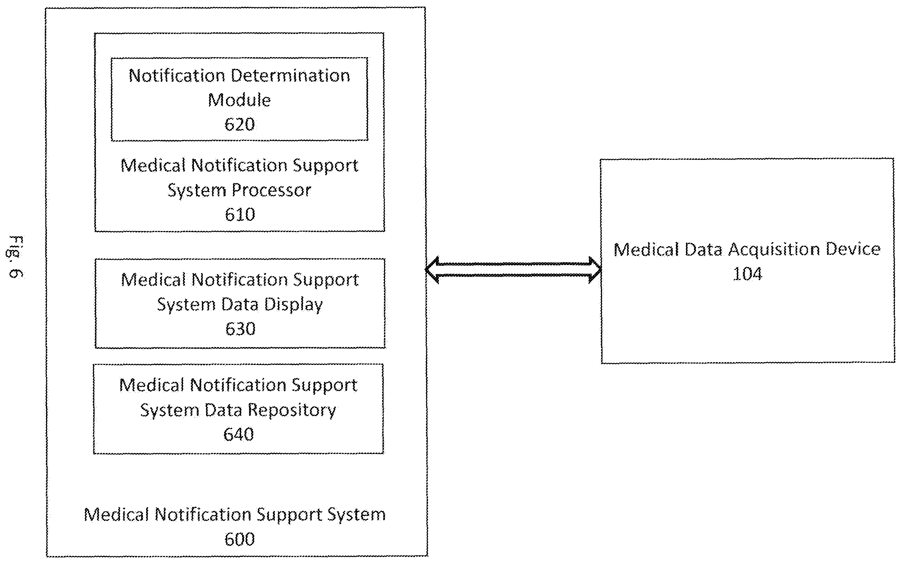

In addition, in response to a diagnosis of a certain medical condition given to another patient 103, a medical notification support system 600 may notify all undiagnosed patients 103, included in the common clusters of the given diagnosis requesting entity 103, of potential infection with the medical conditions, as detailed herein, inter alia Fig. 6.

Attention is now drawn to **Fig. 3****,** showing a block diagram schematically illustrating one example of a medical diagnosis support system, a patient workstation, a medical data acquisition device and a medical practitioner workstation, and various connections therebetween, in accordance with the presently disclosed subject matter.

The medical diagnosis support system 200 can comprise or be otherwise associated with a medical diagnosis support system data repository 320 (e.g. a database, a storage system, a memory including Read Only Memory - ROM, Random Access Memory - RAM, or any other type of memory, etc.) configured to store data, including, inter alia, medical records, diagnoses, medical examinations, etc., as further detailed herein. In some cases, medical diagnosis support system data repository 320 can be further configured to enable retrieval and/or update and/or deletion of the stored data. It is to be noted that in some cases, medical diagnosis support system data repository 320 can be distributed.

The medical diagnosis support system 200 may further include a medical diagnosis support system display 310 (e.g. a computer monitor or any other type of screen or display), capable of displaying information (e.g. displaying diagnosis support information to a medical diagnosing entity 124). It is to be noted that in such cases that the medical diagnosis support system 200 is incorporated into the medical practitioner workstation 122, the medical diagnosis support system display 310 can be the display of the medical practitioner workstation 122.

The medical diagnosis support system 200 may further include a keyboard or any other suitable input/output devices.

The medical diagnosis support system 200 further includes a medical diagnosis support system processor 300. Medical diagnosis support system processor 300 can be one or more processing units (e.g. central processing units), microprocessors, microcontrollers (e.g. microcontroller units (MCUs)) or any other computing devices or modules, including multiple and/or parallel and/or distributed processing units, which are adapted to independently or cooperatively process data for controlling relevant medical diagnosis support system 200 resources and for enabling operations related to medical diagnosis support system 200 resources.

The medical diagnosis support system processor 300 can comprise one or more of the following modules: diagnosis support information management module 330 and a patient queue management module 340.

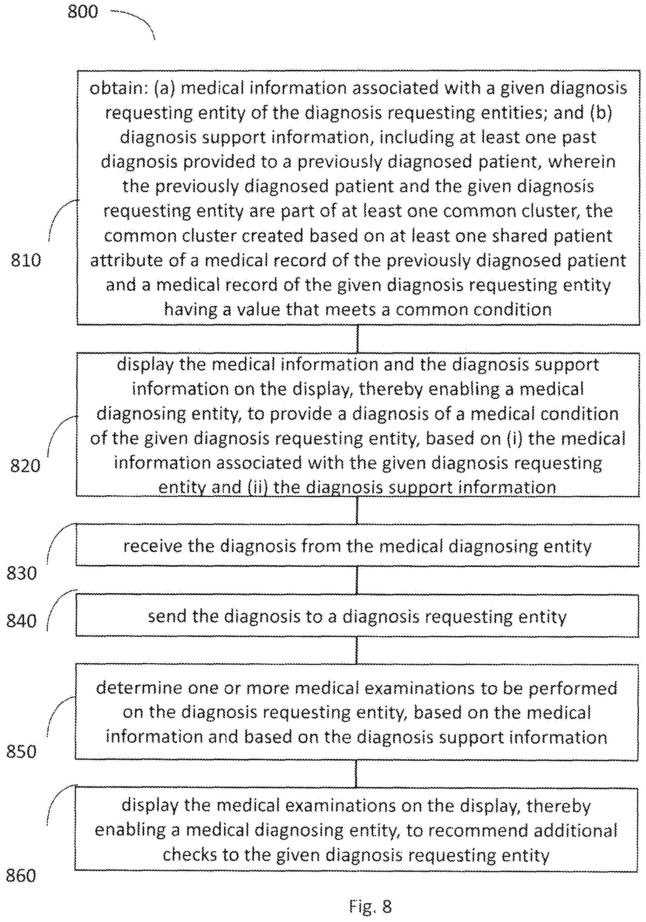

The diagnosis support information management module 330 can be configured to manage the process of obtaining medical information associated with a given diagnosis requesting entity 103, and obtaining diagnosis support information for the given diagnosis requesting entity 103. The diagnosis support information management module 330 can be further configured to display the medical information and the diagnosis support information on the medical diagnosis support system display 310. Displaying such data on the medical diagnosis support system display 310 can enable a medical diagnosing entity 124 to provide a diagnosis of a medical condition of the given diagnosis requesting entity 103, as further detailed, inter alia, with reference to Figs. 8, 12 and 13.

The diagnosis support information management module 330 may be furthered configured to manage the process of determining one or more additional medical examinations to be performed in addition to any examination preformed proir to the diagnosis requesting entity 103 present medical visit and/or examinations comprised within the current check plan of the diagnosis requesting entity 103, in order to obtain additional medical data from the given diagnosis requesting entity 103 and displaying the medical examinations on the medical diagnosis support system display 310. Displaying such data on the medical diagnosis support system display 310 can enable a medical diagnosing entity 124 to recommend additional checks to the given diagnosis requesting entity 103, as further detailed, inter alia, with reference to Fig. 8.

The diagnosis support information management module 330 may be still furthered configured to manage the process of receiving a diagnosis of a medical condition of a given diagnosis requesting entity 103, provided by a medical diagnosing entity 124, from the medical diagnosing entity 124, e.g. via a medical practitioner workstation 122, and sending the diagnosis to the given diagnosis requesting entity 103, e.g. via a patient workstation 114 or via a medical data acquisition device 104, as further detailed, inter alia, with reference to Fig. 8.

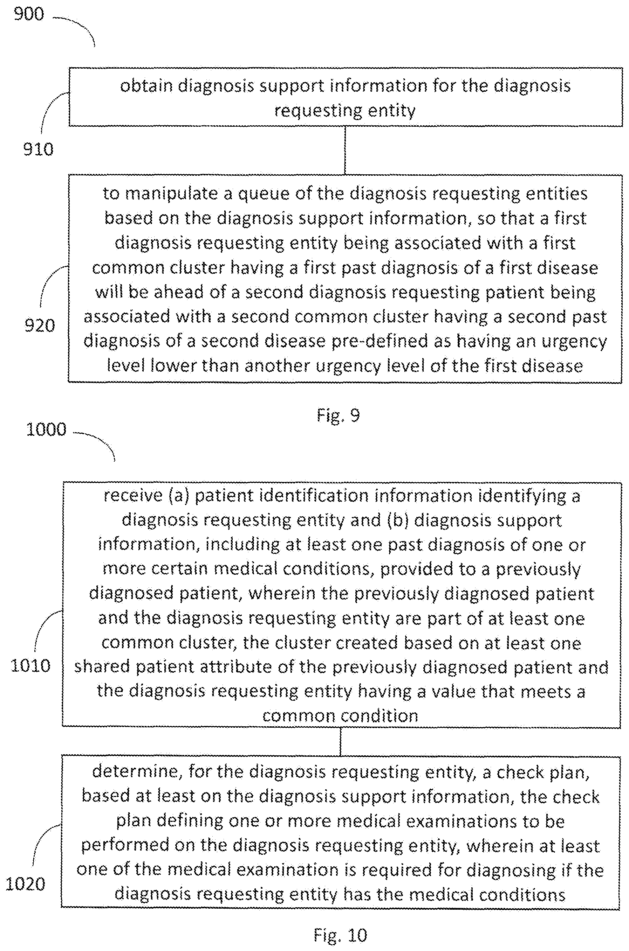

In medicine in general, and in telemedicine in particular, there are various scenarios in which medical diagnosing entities 124 have a queue of diagnosis requesting entities 103, awaiting to be diagnosed, therefore it is beneficial to manipulate the queue of diagnosis requesting entities 103, based on urgency levels, i.e. that emergencies will be handled faster than routine checks. The patient queue management module 340 can be configured to manage the process of manipulating a queue of diagnosis requesting entities 103, so that a first diagnosis requesting entity 103 being associated with a first common cluster having a first past diagnosis of a first disease will be ahead of a second diagnosis requesting entity 103 being associated with a second common cluster having a second past diagnosis of a second disease pre-defined as having an urgency level lower than another urgency level of the first disease, even if the second diagnosis requesting entity 103 entered the queue before the first diagnosis requesting entity 103, as further detailed, inter alia, with reference to Fig. 9. Additionally, or alternatively, patient queue management module 340 can be configured to manage the process of manipulating a queue of diagnosis requesting entities 103, so that a first asthmatic diagnosis requesting entity 103 located at an area with a first air pollution levels will be ahead of a second asthmatic diagnosis requesting entity 103 located at an area with air pollution levels lower than the first air pollution levels.

Attention is now drawn to **Fig. 4****,** showing a block diagram schematically illustrating one example of a medical record management system and a medical diagnosis support system, and various connections therebetween, in accordance with the presently disclosed subject matter.

The medical record management system 210 can comprise or be otherwise associated with a medical record management system data repository 440 (e.g. a database, a storage system, a memory including Read Only Memory - ROM, Random Access Memory - RAM, or any other type of memory, etc.) configured to store data, including, inter alia, medical records, patient's attributes, clusters of medical records, etc., as further detailed herein. In some cases, medical record management system data repository 440 can be further configured to enable retrieval and/or update and/or deletion of the stored data. It is to be noted that in some cases, medical record management system data repository 440 can be distributed.

The medical record management system data repository 440 may hold a plurality of medical records, each medical record is associated with a corresponding patient 103. Each medical record comprises patient identification information (e.g. a patient identification number, patient's biometric identifiers, such as fingerprints, DNA, iris recognition, etc.) which uniquely identifies the corresponding patient 103. In addition, each medical record comprises patient attributes (e.g. patient name, age, age group, address, type of work, place of work, location information, sensitivities to medicines, an identifier of a medical data acquisition device 104 used to acquire medical data therefrom, etc.) of the corresponding patient 103. In addition, at least part of the medical records further includes one or more past diagnosis, previously provided to the corresponding patient 103, e.g. by a medical practitioner 124 of the corresponding patient 103 during a past real (i.e. in-person) or virtual patient visit.

The medical record management system 210 may further include a medical record management system display 430 (e.g. a computer monitor or any other type of screen or display), capable of displaying information (e.g. displaying diagnosis support information to a medical diagnosing entity 124). It is to be noted that in such cases that the medical record management system 210 is incorporated into the medical practitioner workstation 122, the medical record management system display 430 can be the display of the medical practitioner workstation 122.

The medical record management system 210 may further include a keyboard or any other suitable input/output devices.

The medical record management system 210 further includes a medical record management system processor 400. Medical record management system processor 400 can be one or more processing units (e.g. central processing units), microprocessors, microcontrollers (e.g. microcontroller units (MCUs)) or any other computing devices or modules, including multiple and/or parallel and/or distributed processing units, which are adapted to independently or cooperatively process data for controlling relevant medical record management system 210 resources and for enabling operations related to medical record management system 210 resources.

The medical record management system processor 400 can comprise one or more of the following modules: common clusters management module 410 and a diagnosis support management module 420.

The common clusters management module 410 can be configured to manage the process of clustering the medical records into groups, based on the patient attributes. Medical records who share at least one attribute value will be grouped in the same cluster. Where an attribute's value is determined by the medical record management system 210 to be a shared attributes' value, when the value of attributes' value of two or more medical records meets a common condition (e.g. equality of the values, physical proximity of the values, values that are part of a predefined group, etc.), as further detailed, inter alia, with reference to Fig. 7.

The diagnosis support management module 420 can be configured to manage the process of:
(1) Receiving, from a medical diagnosis support system 200, a diagnosis support information request, including the identification information of a given diagnosis requesting entity 103;
(2) Producing diagnosis support information, comprising information of past diagnosis of patient-associated clusters, i.e. clusters that are associated with the medical record of the given diagnosis requesting entity 103; and
(3) Sending, to the medical diagnosis support system 200, a diagnosis support information reply, comprising of the diagnosis support information, i.e. the past diagnosis from the patient-associated clusters, as further detailed, inter alia, with reference to Fig. 7.

Attention is now drawn to **Fig. 5****,** showing a block diagram schematically illustrating one example of a check plan determination system, a medical data acquisition device and a medical diagnosis support system, and various connections therebetween, in accordance with the presently disclosed subject matter.

Environment 20, may include one or more check plan determination systems 500, each capable of determining one or more medical examinations to be performed on a diagnosis requesting entity 103.

In some cases, the check plan determination systems 500 can be located at the medical practitioner location 120. In such cases, the medical record management system 210 may be incorporated into the medical practitioner workstation 122 or may operate as a stand-alone system communicated with via a local network at the medical practitioner location 120. In other cases, the check plan determination systems 500 can be part of central system 130, or it can be a separate system located at the central system 130 location or at other locations. In such cases, the check plan determination systems 500 may communicate with a medical diagnosis support system 200 and/or with a medical record management system 210 and/or with a patient workstation 114 and/or with a medical practitioner workstation 122 and/or with a medical data acquisition device 104 via a communication network 116.

The check plan determination systems 500 may determine, as a result of a given diagnosis request of a diagnosis requesting entity 103 (e.g. via a patient workstation 114 or via medical practitioner workstation 122), a check plan, based at least on diagnosis support information. The diagnosis support information may be obtained by the check plan determination systems 500 itself, or may be obtained from a medical diagnosis support system 200 and/or from a medical record management system 210. The diagnosis support information may include information of past diagnosis of one or more certain medical conditions, provided to a previously diagnosed patient sharing at least one common cluster with the diagnosis requesting entity 103. The common cluster, as indicated herein, is created based on at least one shared patient attribute of the previously diagnosed patient and the given diagnosis requesting entity 103 having a value that meets a common condition (e.g. equality of the values of the attributes, physical proximity of the values of geographical attributes, etc.). For example, in response to a given patient 103 diagnosis request, a check plan is determined for the given patient 103 based on the given patient's past diagnoses common clusters. The determined check plan can include medical examinations whose results can be used in order to identify the conditions characterizing the past diagnoses.

Optionally, the check plan determination process may be initiated in response to a diagnosis given to another patient 103, where such diagnosis requires determining an updated check plan to all other patients in patient's 103 common clusters. For example, in response to a given patient 103 being diagnosed with a contagious disease, a check plan is determined for all other patients 103 on the given patient's common clusters. The determined check plans include a medical examination whose results can be used in order to identify the conditions characterizing the diagnosed contiguous disease.

Optionally, the determined check plan may be uploaded to a medical data acquisition device 104 of the given patient, thus enabling the medical data acquisition device 104 to provide the given patient with instructions for executing the check plan.

The check plan determination system 500 can comprise or be otherwise associated with a check plan determination system data repository 540 (e.g. a database, a storage system, a memory including Read Only Memory - ROM, Random Access Memory - RAM, or any other type of memory, etc.) configured to store data, including, inter alia, check plans, diagnosis requests, diagnosis support information, past diagnoses, etc., as further detailed herein. In some cases, check plan determination system data repository 540 can be further configured to enable retrieval and/or update and/or deletion of the stored data. It is to be noted that in some cases, check plan determination system data repository 540 can be distributed.

The check plan determination system 500 may further include a check plan determination system display 530 (e.g. a computer monitor or any other type of screen or display), capable of displaying information (e.g. displaying diagnosis support information to a medical diagnosing entity 124). It is to be noted that in such cases that the check plan determination system 500 is incorporated into the medical practitioner workstation 122, the check plan determination system display 530 can be the display of the medical practitioner workstation 122.

The check plan determination system 500 may further include a keyboard or any other suitable input/output devices.

The check plan determination system 500 further includes a check plan determination system processor 510. Check plan determination system processor 510 can be one or more processing units (e.g. central processing units), microprocessors, microcontrollers (e.g. microcontroller units (MCUs)) or any other computing devices or modules, including multiple and/or parallel and/or distributed processing units, which are adapted to independently or cooperatively process data for controlling relevant check plan determination system 500 resources and for enabling operations related to check plan determination system 500 resources.

The check plan determination system processor 510 can comprise a check plan determination module 520.

The check plan determination module 520 can be configured to manage the process of check plan determination, as further detailed, inter alia, with reference to Fig. 10.

Attention is now drawn to **Fig. 6****,** showing a block diagram schematically illustrating one example of a medical notification support system and a medical data acquisition device, and various connections therebetween, in accordance with the presently disclosed subject matter.

Environment 20, may further include one or more medical notification support systems 600.

In some cases, the medical notification support systems 600 can be located at the medical practitioner location 120. In such cases, the medical record management system 210 may be incorporated into the medical practitioner workstation 122 or may operate as a stand-alone system communicated with via a local network at the medical practitioner location 120. In other cases, the medical notification support systems 600 can be part of central system 130, or it can be a separate system located at the central system 130 location or at other locations. In such cases, the medical notification support systems 600 may communicate with a medical diagnosis support system 200 and/or with a medical record management system 210 and/or with a patient workstation 114 and/or with a medical practitioner workstation 122 and/or with a medical data acquisition device 104 via a communication network 116.

Each medical notification support systems 600 may obtain notification support information. The notification support information may be automatically determined by the medical notification support systems 600 itself, or may be obtained from a medical diagnosis support system 200 and/or from a medical record management system 210. The notification support information includes at least one past diagnosis of one or more certain medical conditions, provided to a previously diagnosed patient 103, wherein the previously diagnosed patient 103 and one or more undiagnosed patients 103 are part of at least one common cluster, the common cluster created based on at least one shared patient attribute of a medical record of the previously diagnosed patient 103 and a medical record of the undiagnosed patient 103 having a value that meets a common condition (e.g. equality of the values of the attributes, physical proximity of the values of geographical attributes, etc.) and obtain patient identification information identifying the undiagnosed patients 103.

The medical notification support system 600 can comprise or be otherwise associated with a medical notification support system data repository 640 (e.g. a database, a storage system, a memory including Read Only Memory - ROM, Random Access Memory - RAM, or any other type of memory, etc.) configured to store data, including, inter alia, notification support information, etc., as further detailed herein. In some cases, medical notification support system data repository 640 can be further configured to enable retrieval and/or update and/or deletion of the stored data. It is to be noted that in some cases, medical notification support system data repository 640 can be distributed.

The medical notification support system 600 may further include a medical notification support system display 630 (e.g. a computer monitor or any other type of screen or display), capable of displaying information (e.g. displaying diagnosis support information to a medical diagnosing entity 124). It is to be noted that in such cases that the medical notification support system 600 is incorporated into the medical practitioner workstation 122, the medical notification support system display 630 can be the display of the medical practitioner workstation 122.

The medical notification support system 600 may further include a keyboard or any other suitable input/output devices.

The medical notification support systems 600 may further display the notification support information and the patient identification information on the display, thereby enabling a medical diagnosing entity 124, to notify the undiagnosed patients 103 of potential infection with the medical conditions included in the past diagnosis. Optionally, the notification support information may be automatically uploaded by the medical notification support systems 600, without involvement of the medical diagnosing entity 124, to patient workstations 114 and/or to medical data acquisition devices 104 of the undiagnosed patients 103, thus notifying the undiagnosed patients 103 of potential infection with the medical conditions.

The medical notification support system 600 further includes a medical notification support system processor 610. Medical notification support system processor 610 can be one or more processing units (e.g. central processing units), microprocessors, microcontrollers (e.g. microcontroller units (MCUs)) or any other computing devices or modules, including multiple and/or parallel and/or distributed processing units, which are adapted to independently or cooperatively process data for controlling relevant medical notification support system 600 resources and for enabling operations related to medical notification support system 600 resources.

The medical notification support system processor 610 can comprise a notification determination module 620.

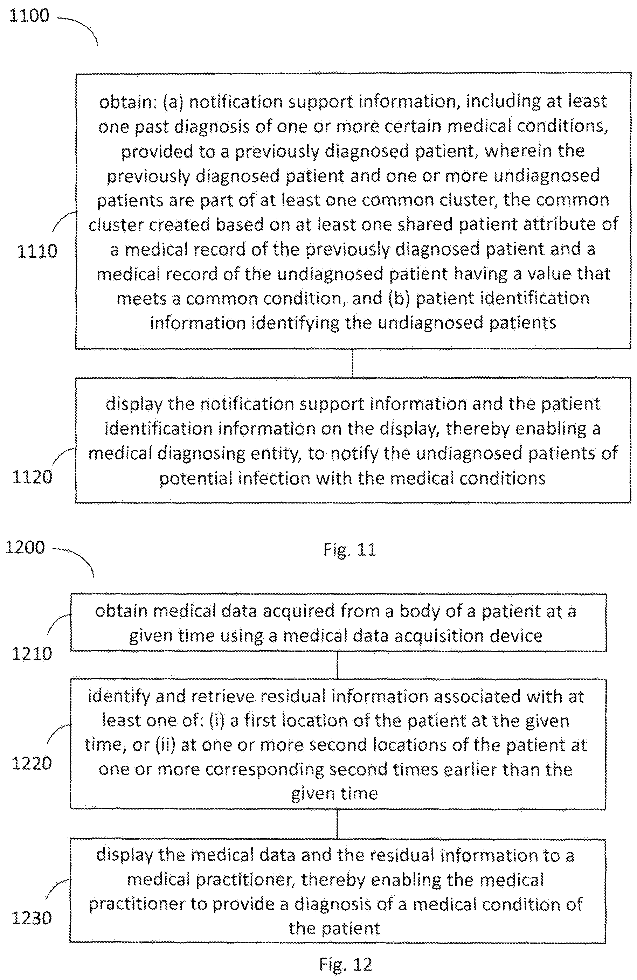

The notification determination module 620 can be configured to manage the process of notification determination, as further detailed, inter alia, with reference to Fig. 11.

Having described the environment 20 and the components thereof, attention is drawn to **Fig. 7****,** showing a flowchart illustrating one example of a sequence of operations carried out by a medical record management system for providing cluster based diagnosis support, in accordance with the presently disclosed subject matter.

According to some examples of the presently disclosed subject matter, medical record management system 210 can be configured to execute a diagnosis support information management process 700, utilizing the common clusters management module 410 and the diagnosis support management module 420.

For this purpose, medical record management system 210 can be configured to provide a plurality of medical records, each associated with a corresponding patient 103, wherein each medical record comprises patient identification information and at least one patient attribute, and wherein one or more of the medical records includes one or more past diagnosis previously provided for the corresponding patient 103. Optionally, the diagnosis support information may include only past diagnosis with calculated likelihood of relevance to the given diagnosis requesting entity 103 that exceeds a certain threshold, e.g. only past diagnosis given in recent timeframe will be included in the diagnosis support information. In addition, the diagnosis support information may include the shared attributes' values, which were the cause for clustering the common clusters associated with diagnosis requesting entity 103 (block 710).

After providing the medical records, the medical record management system 210 can be furthered configured to generate, based on the patient attributes, one or more clusters, each cluster associated with at least two medical records, each having at least one shared patient attribute having a value that meets a common condition (e.g. equality of the values, physical proximity of the values, values that are part of a predefined group, etc.). For example, based on an identical value for the place of work patient attribute, a workplace "X" cluster may be created, holding the medical records of people working at the same workplace "X". In another example, the medical record management system 210 may create a neighborhood "Y" cluster, holding the medical records that have an address value that is within a specific geographical area defining the geographic boundaries of neighborhood "Y". Another example may be based on a physical proximity condition, wherein the medical record management system 210 may create a contiguity "Z" cluster, holding the medical records of all patients 103 having been in physical proximity of each other (e.g. in a location less than 5 meters of each other) during a given time frame (e.g. in the last three days) (block 720).

In parallel to blocks 710 and 720, the medical record management system 210 can be furthered configured to receive a diagnosis support information request, including the identification information of a given patient 103 (block 730).

After receiving the diagnosis support information request, the medical record management system 210 can be furthered configured to identify, using the identification information of the given patient 103, one or more patient-associated clusters of the clusters each associated with the medical record of the given patient 103, wherein at least one of the medical records of each patient-associated cluster, other than the medical record of the given patient 103, includes one or more of the past diagnosis (block 740).

After identifying the patient-associated clusters, the medical record management system 210 can be furthered configured to send a diagnosis support information reply, including the past diagnoses, and optionally only relevant past diagnoses, of the patient-associated clusters (block 750).

It is to be noted that, with reference to Fig. 7, some of the blocks can be integrated into a consolidated block or can be broken down to a few blocks and/or other blocks may be added. Furthermore, in some cases, the blocks can be performed in a different order than described herein (for example, block 730 can be performed before block 720, etc.). It is to be further noted that some of the blocks are optional. It should be also noted that whilst the flow diagram is described also with reference to the system elements that realizes them, this is by no means binding, and the blocks can be performed by elements other than those described herein.

Attention is now drawn to **Fig. 8****,** showing a flowchart illustrating one example of a sequence of operations carried out by a medical diagnosis support system for providing cluster based diagnosis support, in accordance with the presently disclosed subject matter.

According to some examples of the presently disclosed subject matter, medical diagnosis support system 200 can be configured to execute a diagnosis support information management process 800, utilizing the diagnosis support information management module 330.

For this purpose, medical diagnosis support system 200 can be configured to obtain medical information associated with a given diagnosis requesting entity 103. The medical information may be obtained from a medical diagnosing entity 124 and/or from diagnosis requesting entity 103. The medical diagnosis support system 200 can be furthered configured to obtain diagnosis support information for the given diagnosis requesting entity 103. The diagnosis support information may be obtained by the medical diagnosis support system 200 itself, for example, by determining the diagnosis support information based on clustering of medical records of diagnosis requesting entities 103, stored in the medical diagnosis support system data repository 320. The medical diagnosis support system 200 may cluster the medical records into groups, based on the patient attributes. Medical records who share attributes' values will be grouped in the same cluster. Where an attribute's value is determined by the medical diagnosis support system 200 to be a shared attributes' value, when the value of attributes' value of two or more medical records meets a common condition (e.g. equality of the values, physical proximity of the values, values that are part of a predefined group, etc.). The diagnosis support information may be optionally obtained by the medical diagnosis support system 200 from a medical record management system 210, as detailed above, inter alia Fig. 4.

The diagnosis support information includes at least one past diagnosis provided to a previously diagnosed patient, wherein the previously diagnosed patient and the given diagnosis requesting entity 103 are part of at least one common cluster, as detailed above, with respect to Fig. 2. Optionally, the diagnosis support information may include only past diagnoses with calculated likelihood of relevance to the given diagnosis requesting entity 103 that exceeds a certain threshold, e.g. only past diagnosis given in recent timeframe will be included in the diagnosis support information. For example, only past diagnoses provided to previously diagnosed patients in the last 5 days, will be included in the diagnosis support information. In addition, the timeframe may be calculated depending on the specific medical condition diagnosed in the past diagnoses, e.g. a past diagnosis of a disease with a continuing effect will have a longer timeframe than a disease with a transitory effect. For example, a past diagnosis of HIV will have a long timeframe of relevance (optionally an infinite timeframe), while a past diagnosis of Flu will have a short timeframe of relevance (e.g. one week).

In addition, the diagnosis support information may include the shared patient attributes' values, which were the cause for clustering the common clusters associated with the given diagnosis requesting entity 103. Shared patient attribute may include: name, address, workplace address, location information (e.g. obtained from a GPS of a smartphone of the diagnosis requesting entity 103), with the corresponding values for the given diagnosis requesting entity 103. The shared patient attributes' values may be, for example, a value of workplace "X" for the place of work attribute, thus the diagnosis support information will include, in addition to past diagnosis given to members of the workplace "X" cluster, also the value of "X" for the place of work attribute (block 810).

After obtaining the medical information and the diagnosis support information, medical diagnosis support system 200 may be further configured to display the medical information and the diagnosis support information on a medical diagnosis support system display 310 (e.g. a computer monitor or any other type of screen or display). It is to be noted that in such cases that the medical diagnosis support system 200 is incorporated into the medical practitioner workstation 122, the medical information and the diagnosis support information are displayed on a display of the medical practitioner workstation 122. Displaying the medical information and the diagnosis support information enables the medical diagnosing entity 124, to provide a diagnosis of a medical condition of the given diagnosis requesting entity 103 based on the medical information and the diagnosis support information (block 820).

The medical diagnosis support system 200 may be further configured to receive a diagnosis of a medical condition of a given diagnosis requesting entity 103, provided by a medical diagnosing entity 124, e.g. via a medical practitioner workstation 122 (block 830).

After receiving the diagnosis of a medical condition of a given diagnosis requesting entity 103, the medical diagnosis support system 200 may be further configured to send the diagnosis to the given diagnosis requesting entity 103, e.g. via a patient workstation 114 or via a medical data acquisition device 104, or in any other manner (block 840).

Additionally, or alternatively to block 820, after obtaining the medical information and the diagnosis support information, the medical diagnosis support system 200 may be configured to determine one or more additional medical examinations to be performed in order to obtain additional medical data from a certain diagnosis requesting entity 103. The additional medical examinations are determined based on the medical information and based on the diagnosis support information. For example, based on the diagnosis support information, which includes one or more past diagnoses of a certain contiguous disease in the cluster of a given diagnosis requesting entity 103, the medical diagnosis support system 200 may determine a set of suggested additional examinations, relevant to diagnosing such contiguous disease (block 850).

After determining the additional medical examinations, the medical diagnosis support system 200 may be further configured to display the additional medical examinations on the medical diagnosis support system display 310 (e.g. a computer monitor or any other type of screen or display). It is to be noted that in such cases that the medical diagnosis support system 200 is incorporated into the medical practitioner workstation 122, the additional medical examinations are displayed on a medical practitioner workstation 122. Displaying the additional medical examinations hereby can enable the medical diagnosing entity 124 to recommend additional medical examinations to be performed on the diagnosis requesting entity 103, e.g. a list of suggested additional examinations are displayed to the medical diagnosing entity 124 and the medical diagnosing entity 124 can decide whether to instruct/recommend the diagnosis requesting entity 103 to perform one or more of the additional examinations. For example, based on the diagnosis support information, which includes a few past diagnoses of a certain contiguous disease in the cluster of a given diagnosis requesting entity 103, the medical diagnosis support system 200 may display to the medical diagnosing entity 124, on the display of the medical practitioner workstation 122, a set of suggested additional examinations, relevant to diagnosing the contiguous disease. The medical diagnosing entity 124 can decide whether to instruct/recommend the diagnosis requesting entity 103 to perform one or more of these additional examinations.

Optionally, information of the required additional medical examinations can be automatically sent to a medical data acquisition device 104 of the diagnosis requesting entity 103, thereby enabling it to instruct the diagnosis requesting entity 103 to perform the additional medical examinations utilizing the medical data acquisition device 104, i.e. with no need to manually set-up the medical data acquisition device 104 to be suitable for the performance of the additional medical examinations.

Alternatively, the additional medical examination can be introduced into a check plan (stored on a patient & check plan repository 136) associated with the diagnosis requesting entity 103, so that the next time the medical data acquisition device 104 is used to collect medical data from the diagnosis requesting entity 103, it will also perform the additional medical examination.

Continuing the example above, the additional medical examinations selected by the medical diagnosing entity 124, in order to diagnose the contiguous disease, may be automatically sent to the medical data acquisition device 104 of the diagnosis requesting entity 103, thereby enabling it to instruct the diagnosis requesting entity 103 to complete the additional medical examinations with the medical data acquisition device 104. Additionally, or alternatively, the additional medical examinations may be added to the check plan associated with the diagnosis requesting entity 103, stored on the patient & check plan repository 136. In such cases, the next time the medical data acquisition device 104 is used to collect medical data from the diagnosis requesting entity 103, it will also perform the additional medical examination/s (block 860).

It is to be noted that, with reference to Fig. 8, some of the blocks can be integrated into a consolidated block or can be broken down to a few blocks and/or other blocks may be added. Furthermore, in some cases, the blocks can be performed in a different order than described herein (for example, block 850 can be performed before block 820, etc.). It is to be further noted that some of the blocks are optional. It should be also noted that whilst the flow diagram is described also with reference to the system elements that realizes them, this is by no means binding, and the blocks can be performed by elements other than those described herein.

Turning to **Fig. 9****,** a flowchart is provided, illustrating one example of a sequence of operations carried out by a medical diagnosis support system for manipulate a queue of the diagnosis requesting entities, in accordance with the presently disclosed subject matter.

According to some examples of the presently disclosed subject matter, medical diagnosis support system 200 can be configured to execute a patient queue management process 900, utilizing the patient queue management module 340.

In medicine in general, and in telemedicine in particular, there are numerous scenarios in which medical diagnosing entities 124 have a queue of diagnosis requesting entities 103, awaiting to be diagnosed, either by physically waiting at the medical practitioner location 120 or by sending requests for diagnosis to a medical practitioner workstation 122. Therefore, it is beneficial to manipulate the queue of diagnosis requesting entities 103, based on urgency levels, i.e. that emergencies will be handled faster than routine checks.

For this purpose, medical diagnosis support system 200 can be configured to obtain medical information associated with each diagnosis requesting entities 103 within a queue, awaiting to be diagnosed. The medical information may be obtained from a medical diagnosing entity 124 and/or from diagnosis requesting entity 103. The medical diagnosis support system 200 can be further configured to obtain diagnosis support information for the given diagnosis requesting entity 103. The diagnosis support information may be obtained by the medical diagnosis support system 200 itself, for example, by calculating the diagnosis support information based on medical records stored in the medical diagnosis support system data repository 320. The diagnosis support information may be optionally obtained from a medical record management system 210, all as detailed above, inter alia block 810 of Fig. 8 (block 910).

After obtaining the medical information and the diagnosis support information, associated with each diagnosis requesting entity 103 within the queue of diagnosis requesting entities 103 awaiting to be diagnosed, medical diagnosis support system 200 may be further configured to manipulate the queue of the diagnosis requesting entities 103, if required. The manipulation is based on the diagnosis support information, so that a first diagnosis requesting entity 103 being associated with a first common cluster having a first past diagnosis of a first disease will be ahead of a second diagnosis requesting entity 103 being associated with a second common cluster having a second past diagnosis of a second disease pre-defined as having an urgency level lower than another urgency level of the first disease, even if the second diagnosis requesting entity 103 entered the queue before the first diagnosis requesting entity 103.

In a specific example, if a first diagnosis requesting entity 103, being associated with a specific cluster having a past diagnosis of a disease with a low urgency level (e.g. common flu), and a second diagnosis requesting entity 103, being associated with a specific cluster having a past diagnosis of a disease with a high urgency level (e.g. Ebola), are awaiting to receive a diagnosis from a given medical diagnosing entity 124, than the medical diagnosis support system 200 may make sure that the second diagnosis requesting entity 103 is ahead of the first diagnosis requesting entity 103 in the queue to receive diagnosis from the given medical diagnosing entity 124, even if the first diagnosis requesting entity 103 entered the queue before the second diagnosis requesting entity 103.

In another example, a diagnosis requesting entity 103, being associated with a specific cluster having a past diagnosis of an infectious disease is advanced over a diagnosis requesting entity 103, being associated with a specific cluster having a past diagnosis of a non-infectious disease (block 920).

It is to be noted that, with reference to Fig. 9, some of the blocks can be integrated into a consolidated block or can be broken down to a few blocks and/or other blocks may be added. It should be also noted that whilst the flow diagram is described also with reference to the system elements that realizes them, this is by no means binding, and the blocks can be performed by elements other than those described herein.

Attention is now drawn to **Fig. 10****,** showing a flowchart illustrating one example of a sequence of operations carried out by a check plan determination system for providing cluster based diagnosis support, in accordance with the presently disclosed subject matter.

According to some examples of the presently disclosed subject matter, check plan determination system 500 can be configured to execute a check plan determination process 1000, utilizing the check plan determination module 520.

For this purpose, check plan determination system 500 can be configured to receive patient identification information identifying a diagnosis requesting entity 103 and to receive diagnosis support information. The diagnosis support information including at least one past diagnosis of one or more certain medical conditions, provided to a previously diagnosed patient 103, wherein the previously diagnosed patient 103 and the diagnosis requesting entity 103 are part of at least one common cluster. The common clusters created based on at least one shared patient attribute of the previously diagnosed patient 103 and the diagnosis requesting entity 103 having a value that meets a common condition (block 1010).

After receiving the patient identification information and the diagnosis support information, the check plan determination system 500 can be further configured to determine, for the diagnosis requesting entity 103, a check plan, based at least on the diagnosis support information. The check plan defines one or more medical examinations to be performed on the diagnosis requesting entity 103, wherein at least one of the medical examination is required for diagnosing if the diagnosis requesting entity 103 has the medical conditions that were previously diagnosed for at least one other diagnosis requesting entity 103 that is part of the common the cluster (block 1020).

It is to be noted that, with reference to Fig. 10, some of the blocks can be integrated into a consolidated block or can be broken down to a few blocks and/or other blocks may be added. It should be also noted that whilst the flow diagram is described also with reference to the system elements that realizes them, this is by no means binding, and the blocks can be performed by elements other than those described herein.

Attention is drawn to **Fig. 11****,** showing a flowchart illustrating one example of a sequence of operations carried out by a medical notification support system for providing cluster based notification support, in accordance with the presently disclosed subject matter.

According to some examples of the presently disclosed subject matter, medical notification support system 600 can be configured to execute a notification determination process 1100, utilizing the notification determination module 620.

For this purpose, medical notification support system 600 can be configured to obtain notification support information. The notification support information includes at least one past diagnosis of one or more certain medical conditions, provided to a previously diagnosed patient 103, wherein the previously diagnosed patient 103 and one or more undiagnosed patients 103 are part of at least one common cluster. The common cluster created based on at least one shared patient attribute of a medical record of the previously diagnosed patient 103. Additionally, the notification support information includes a medical record of the undiagnosed patient 103, having a value that meets a common condition. In addition, the medical notification support system 600 can be configured to obtain patient identification information identifying the undiagnosed patients 103 (block 1110).

After obtaining the notification support information and the patient identification information, the medical notification support system 600 can be further configured to display the notification support information and the patient identification information on the medical notification support system data display 630, thereby enabling a medical diagnosing entity 124, to notify the undiagnosed patients 103 of potential infection with the medical conditions. Optionally, the medical notification support system 600 may automatically notify the undiagnosed patients 103 of potential infection with the medical conditions, directly, without involving the medical diagnosing entity 124, via patient workstations 114 and/or via medical data acquisition devices 104 (block 1120).

It is to be noted that, with reference to Fig. 11, some of the blocks can be integrated into a consolidated block or can be broken down to a few blocks and/or other blocks may be added. It should be also noted that whilst the flow diagram is described also with reference to the system elements that realizes them, this is by no means binding, and the blocks can be performed by elements other than those described herein.

Turning to **Fig. 12****,** there is shown a flowchart illustrating one example of a sequence of operations carried out for providing residual information to a medical practitioner, in accordance with the presently disclosed subject matter.

According to some examples of the presently disclosed subject matter, medical diagnosis support system 200 can be configured to execute another diagnosis support information management process 1200, utilizing the diagnosis support information management module 330.

For this purpose, medical diagnosis support system 200 is configured to obtain medical data relating to a patient 103 (block 1210). The medical data can be acquired from a body of the patient 103 at a given time, e.g. using medical data acquisition device 104, or any other suitable device capable of acquiring medical data from a body of the patient 103, or it can be obtained in any other manner (including for example by the patient 103 providing the information by answering a questionnaire).

When using the the medical data acquisition device 104, which includes at least one one medical data acquisition sensor 106, the medical data can include at least one measurement obtained by the medical data acquisition sensor 106.

Medical diagnosis support system 200 is further configured to identify and retrieve residual information associated with at least one of: (i) a location of the patient 103 at the time at which the medical data is obtained at block 1210, or (ii) at one or more other locations of the patient 103 at one or more corresponding times earlier than the time at which the medical data is obtained at block 1210 (block 1220).

The residual information can include one or more of: one or more air pollution indicators indicative of current air pollution levels at the current location of the patient 103, and/or past air pollution levels at locations that the patient visited at respective times up to a certain time before the time at which the medical data is obtained at block 1210; one or more water pollution indicators indicative of current water pollution levels at the current location of the patient 103, and/or past water pollution levels at locations that the patient visited at respective times up to a certain time before the time at which the medical data is obtained at block 1210; information of a current disease outbreak at the current location of the patient 103, and/or a past disease outbreak at locations that the patient visited at respective times up to a certain time before the time at which the medical data is obtained at block 1210; information of a current radiation level at the current location of the patient 103, and/or a past radiation level at locations that the patient visited at respective times up to a certain time before the time at which the medical data is obtained at block 1210; current weather information indicative of the weather at the current location of the patient 103, and/or past weather information at locations that the patient visited at respective times up to a certain time before the time at which the medical data is obtained at block 1210; food poisoning information; current known diseases at the current location of the patient 103, and/or past known diseases at locations that the patient visited at respective times up to a certain time before the time at which the medical data is obtained at block 1210; presence of the patient 103 on a flight; participation of the patient 103 in scuba diving; etc.

The retrieved residual information is identified using a rule set defining a relevance of the residual information for diagnosis purposes. Clearly, it is desirable to identify residual information that has value for diagnosis purposes. The fact that at a given location there was an outbreak of Flu three months before a patient 103 complained about Flu-related symptoms, is irrelevant for providing diagnosis for such patient 103. On the contrary, if at the same given location there was an outbreak of Flu a day before the patient 103 complained about Flu-related symptoms, clearly the fact that such a Flu outbreak was identified at that time is extremely relevant for providing diagnosis for such patient 103, which most likely has Flu.

Similarly, if unusually high air pollution is measured at a certain location of a given asthmatic patient 103 two days before the asthmatic patient 103 complains about asthma-related symptoms - having such knowledge is irrelevant as it is not indicative of a potential cause for the patient 103 to have the asthma-related symptoms, whereas if the unusually high air pollution is measured at the certain location of the given asthmatic patient 103 an hour before she complains about asthma-related symptoms - having such knowledge is extremely relevant for providing diagnosis for such patient 103, which most likely experiences the asthma-related symptoms due to current high air pollution.

The rule set based on which relevance can be determined is based on at least one of: (a) the time at which the medical data is obtained at block 1210, the location of the patient at the time at which the medical data is obtained at block 1210 and metadata defining a time span of relevance of types of residual information (e.g. for Flu the relevant time span is a few days, for air pollution the relevant time span is one day, etc.), (b) locations of the patient 103 before the medical data is obtained at block 1210 and the respective times and metadata defining a time span of relevance of types of residual information (e.g. for Flu the relevant time span is a few days, for air pollution the relevant time span is one day, etc.), (c) known medical conditions of the patient (if the patient 103 is known to have asthma, air pollution levels are of high relevance when attempting to diagnose a medical condition thereof), or (d) the acquired medical data (if the medical data indicates that the patient's 103 suffers from shortness of breath, also known as dyspnea, air pollution levels are of high relevance when attempting to diagnose a medical condition thereof).

In some cases, the residual information is obtained from on-line sources, such as the Internet (e.g. from websites providing information on air pollution, weather, water pollution, disease outbreaks, etc.). In some cases, at least one of the on-line sources is external to the medical diagnosis support system 200. In other cases, the residual information can be obtained from local authorities and/or from international bodies that collect such information.

Medical diagnosis support system 200 displays the medical data and the residual information to a medical practitioner, thereby enabling the medical practitioner to provide a diagnosis of a medical condition of the patient (block 1230).

In some cases, the medical data is acquired from the patient body and displayed to the medical practitioner during an on-line session between the patient and the medical practitioner. In other cases, the medical data is acquired from the patient body at a given time and displayed to the medical practitioner at a later time, later than the given time. This can be the case for example when the medical data acquisition device 104 is not in real-time communication with the medical diagnosis support system 200.

It is to be noted that, with reference to Fig. 12, some of the blocks can be integrated into a consolidated block or can be broken down to a few blocks and/or other blocks may be added. It should be also noted that whilst the flow diagram is described also with reference to the system elements that realizes them, this is by no means binding, and the blocks can be performed by elements other than those described herein.

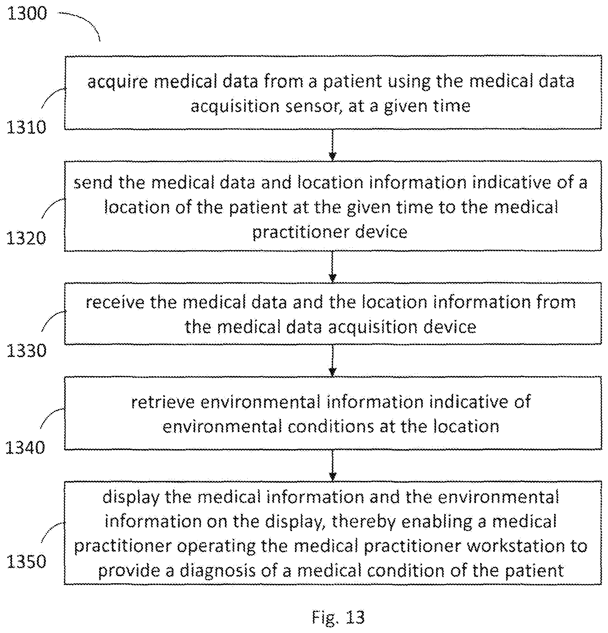

Turning to **Fig. 13****,** there is shown a flowchart illustrating another example of a sequence of operations carried out for providing residual information to a medical practitioner, in accordance with the presently disclosed subject matter.

According to some examples of the presently disclosed subject matter, medical data acquisition device 104 and medical diagnosis support system 200 can be configured to execute another diagnosis support information management process 1200.

For this purpose, medical data acquisition device 104 can be configured to acquire medical data from a patient 103 using the medical data acquisition sensor 106, at a given time (block 1310), and to send the medical data and location information indicative of a location of the patient 103 at the given time to the medical diagnosis support system 200 (block 1320).

The medical diagnosis support system 200 receives the medical data and the location information from the medical data acquisition device 104 (block 1330), retrieves (similarly to block 1220) environmental information indicative of environmental conditions at the location (block 1340), and displays the medical information and the environmental information on the display, thereby enabling a medical practitioner operating the medical practitioner workstation to provide a diagnosis of a medical condition of the patient (block 1350).

It is to be noted that, with reference to Fig. 13, some of the blocks can be integrated into a consolidated block or can be broken down to a few blocks and/or other blocks may be added. It should be also noted that whilst the flow diagram is described also with reference to the system elements that realizes them, this is by no means binding, and the blocks can be performed by elements other than those described herein.

It is to be understood that the presently disclosed subject matter is not limited in its application to the details set forth in the description contained herein or illustrated in the drawings. The presently disclosed subject matter is capable of other embodiments and of being practiced and carried out in various ways. Hence, it is to be understood that the phraseology and terminology employed herein are for the purpose of description and should not be regarded as limiting. As such, those skilled in the art will appreciate that the conception upon which this disclosure is based may readily be utilized as a basis for designing other structures, methods, and systems for carrying out the several purposes of the present presently disclosed subject matter.

It will also be understood that the system according to the presently disclosed subject matter can be implemented, at least partly, as a suitably programmed computer. Likewise, the presently disclosed subject matter contemplates a computer program being readable by a computer for executing the disclosed method. The presently disclosed subject matter further contemplates a machine-readable memory tangibly embodying a program of instructions executable by the machine for executing the disclosed method.

## Claims

1. A medical diagnosis support system (200), the medical diagnosis support system (200) comprising a processing resource configured to:
obtain medical data acquired from a body of a patient at a given time using a medical data acquisition device (104);
identify and retrieve residual information associated with at least one of: (i) a first location of the patient (103) at the given time, or (ii) at one or more second locations of the patient (103) at one or more corresponding second times earlier than the given time; wherein the retrieved residual information is identified using a first rule set defining a relevance of the residual information for diagnosis purposes based on at least one of: (a) the first location and the given time and metadata defining a time span of relevance of types of the residual information, or (b) the second locations, the corresponding second times and the metadata defining the time span of relevance of types of the residual information; and
display the medical data and the residual information to a medical practitioner (124), thereby enabling the medical practitioner (124) to provide a diagnosis of a medical condition of the patient.

2. The medical diagnosis support system (200) of claim 1, wherein the first rule set defines the relevance of the residual information for diagnosis purposes also based on at least one of: (a) known medical conditions of the patient (103), or (b) the acquired medical data.

3. The medical diagnosis support system (200) of claim 1, wherein the medical data is acquired from the patient (103) body and displayed to the medical practitioner (124) during an on-line session between the patient (103) and the medical practitioner (124).

4. The medical diagnosis support system (200) of claim 1, wherein the medical data is acquired from the patient (103) body and displayed to the medical practitioner (124) at a third time, after the given time, wherein the medical data acquisition device (104) is not in communication with the medical diagnosis support system (200).

5. The medical diagnosis support system (200) of claim 1, wherein the residual information includes one or more of:
a. one or more air pollution indicators;
b. one or more water pollution indicators;
c. information of a disease outbreak;
d. information of a radiation level;
e. weather information;
f. food poisoning information; or
g. known diseases at the first location or at the second locations.

6. The medical diagnosis support system (200) of claim 1, wherein the residual information is obtained from on-line sources, wherein at least one of the on-line sources is external to the medical diagnosis support system (200).

7. The medical diagnosis support system (200) of claim 1, wherein the medical data acquisition device (104) includes at least one one medical data acquisition sensor (106), and wherein the medical data includes at least one measurement obtained by the medical data acquisition sensor (106).

8. A medical diagnosis support method, the method comprising:
obtaining medical data acquired from a body of a patient (103) at a given time using a medical data acquisition device (104);
identifying and retrieving residual information associated with at least one of: (i) a first location of the patient (103) at the given time, or (ii) at one or more second locations of the patient (103) at one or more corresponding second times earlier than the given time; wherein the retrieved residual information is identified using a first rule set defining a relevance of the residual information for diagnosis purposes based on at least one of: (a) the first location and the given time and metadata defining a time span of relevance of types of the residual information, or (b) the second locations, the corresponding second times and the metadata defining the time span of relevance of types of the residual information; and
displaying the medical data and the residual information to a medical practitioner (124), thereby enabling the medical practitioner (124) to provide a diagnosis of a medical condition of the patient (103).

9. The medical diagnosis support method of claim 8, wherein the first rule set defines the relevance of the residual information for diagnosis purposes also based on at least one of: (a) known medical conditions of the patient (103), or (b) the acquired medical data.

10. The medical diagnosis support method of claim 8, wherein the medical data is acquired from the patient (103) body and displayed to the medical practitioner (124) during an on-line session between the patient (103) and the medical practitioner (124).

11. The medical diagnosis support method of claim 8, wherein the medical data is acquired from the patient (103) body and displayed to the medical practitioner (124) at a third time, after the given time, wherein the medical data acquisition device (104) is not in communication with a medical diagnosis support system (200).

12. The medical diagnosis support method of claim 8, wherein the residual information includes one or more of:
a. one or more air pollution indicators;
b. one or more water pollution indicators;
c. information of a disease outbreak;
d. information of a radiation level;
e. weather information;
f. food poisoning information; or
g. known diseases at the first location or at the second locations.

13. The medical diagnosis support method of claim 8, wherein the residual information is obtained from on-line sources, wherein at least one of the on-line sources is external to a medical diagnosis support system (200).

14. The medical diagnosis support method of claim 8, wherein the medical data acquisition device (104) includes at least one one medical data acquisition sensor (106), and wherein the medical data includes at least one measurement obtained by the medical data acquisition sensor (106).

15. A non-transitory computer readable storage medium having computer readable program code embodied therewith, the computer readable program code, executable by at least one processor of a computer to perform a method comprising:
obtaining medical data acquired from a body of a patient (103) at a given time using a medical data acquisition device (104);
identifying and retrieving residual information associated with at least one of: (i) a first location of the patient (103) at the given time, or (ii) at one or more second locations of the patient (103) at one or more corresponding second times earlier than the given time; wherein the retrieved residual information is identified using a first rule set defining a relevance of the residual information for diagnosis purposes based on at least one of: (a) the first location and the given time and metadata defining a time span of relevance of types of the residual information, or (b) the second locations, the corresponding second times and the metadata defining the time span of relevance of types of the residual information; and
displaying the medical data and the residual information to a medical practitioner (124), thereby enabling the medical practitioner (124) to provide a diagnosis of a medical condition of the patient (103).

## Patentansprüche

1. Medizinisches Diagnoseunterstützungssystem (200), wobei das medizinische Diagnoseunterstützungssystem (200) eine Verarbeitungsressource umfasst, konfiguriert zum:
Erhalten medizinischer Daten, die zu einem gegebenen Zeitpunkt mit Hilfe eines medizinischen Datenerfassungsgeräts (104) von einem Körper eines Patienten erfasst werden;
Identifizieren und Abrufen von Restinformationen, die mit mindestens einem assoziiert sind von: (i) einem ersten Standort des Patienten (103) zu dem gegebenen Zeitpunkt oder (ii) an einem oder mehreren zweiten Standorten des Patienten (103) zu einem oder mehreren entsprechenden zweiten Zeitpunkten, die vor dem gegebenen Zeitpunkt liegen; wobei die abgerufenen Restinformationen anhand eines ersten Regelsatzes identifiziert werden, der eine Relevanz der Restinformationen für Diagnosezwecke definiert auf der Basis von mindestens einem von: (a) dem ersten Standort und dem gegebenen Zeitpunkt und Metadaten, die einen Zeitraum der Relevanz von Typen der Restinformationen definieren, oder (b) den zweiten Standorten, den entsprechenden zweiten Zeitpunkten und den Metadaten, die den Zeitraum der Relevanz von Typen der Restinformationen definieren; und
Anzeigen der medizinischen Daten und der Restinformationen einem Arzt (124), so dass der Arzt (124) eine Diagnose eines medizinischen Zustands des Patienten stellen kann.

2. Medizinisches Diagnoseunterstützungssystem (200) nach Anspruch 1, wobei der erste Regelsatz die Relevanz der Restinformationen für Diagnosezwecke auch auf der Basis von mindestens einem von: (a) bekannten medizinischen Zuständen des Patienten (103) oder (b) den erfassten medizinischen Daten definiert.

3. Medizinisches Diagnoseunterstützungssystem (200) nach Anspruch 1, wobei die medizinischen Daten vom Körper des Patienten (103) erfasst und dem Arzt (124) während einer Online-Sitzung zwischen dem Patienten (103) und dem Arzt (124) angezeigt werden.

4. Medizinisches Diagnoseunterstützungssystem (200) nach Anspruch 1, wobei die medizinischen Daten vom Körper des Patienten (103) erfasst und dem Arzt (124) zu einem dritten Zeitpunkt nach dem gegebenen Zeitpunkt angezeigt werden, wobei das medizinische Datenerfassungsgerät (104) nicht mit dem medizinischen Diagnoseunterstützungssystem (200) in Verbindung steht.

5. Medizinisches Diagnoseunterstützungssystem (200) nach Anspruch 1, wobei die Restinformationen eines oder mehrere beinhalten von:
a. einem oder mehreren Luftverschmutzungsindikatoren;
b. einem oder mehreren Wasserverschmutzungsindikatoren;
c. Informationen über einen Krankheitsausbruch;
d. Informationen über eine Strahlungsbelastung;
e. Wetterinformationen;
f. Informationen über Lebensmittelvergiftungen; oder
g. bekannten Krankheiten am ersten Standort oder an den zweiten Standorten.

6. Medizinisches Diagnoseunterstützungssystem (200) nach Anspruch 1, wobei die Restinformationen aus Online-Quellen erhalten werden, wobei mindestens eine der Online-Quellen außerhalb des medizinischen Diagnoseunterstützungssystems (200) liegt.

7. Medizinisches Diagnoseunterstützungssystem (200) nach Anspruch 1, wobei das medizinische Datenerfassungsgerät (104) mindestens einen medizinischen Datenerfassungssensor (106) umfasst und wobei die medizinischen Daten mindestens einen vom medizinischen Datenerfassungssensor (106) erhaltenen Messwert umfassen.

8. Medizinisches Diagnoseunterstützungsverfahren, wobei das Verfahren Folgendes beinhaltet:
Erhalten medizinischer Daten, die zu einem gegebenen Zeitpunkt mit Hilfe eines medizinischen Datenerfassungsgeräts (104) von einem Körper eines Patienten (103) erfasst werden;
Identifizieren und Abrufen von Restinformationen, die mit mindestens einem assoziiert sind von: (i) einem ersten Standort des Patienten (103) zu dem gegebenen Zeitpunkt oder (ii) an einem oder mehreren zweiten Standorten des Patienten (103) zu einem oder mehreren entsprechenden zweiten Zeitpunkten, die vor dem gegebenen Zeitpunkt liegen; wobei die abgerufenen Restinformationen anhand eines ersten Regelsatzes identifiziert werden, der eine Relevanz der Restinformationen für Diagnosezwecke definiert auf der Basis von mindestens einem von: (a) dem ersten Standort und dem gegebenen Zeitpunkt und Metadaten, die einen Zeitraum der Relevanz von Typen der Restinformationen definieren, oder (b) den zweiten Standorten, den entsprechenden zweiten Zeitpunkten und den Metadaten, die den Zeitraum der Relevanz von Typen der Restinformationen definieren; und
Anzeigen der medizinischen Daten und der Restinformationen einem Arzt (124), so dass der Arzt (124) eine Diagnose eines medizinischen Zustands des Patienten (103) stellen kann.

9. Medizinisches Diagnoseunterstützungsverfahren nach Anspruch 8, wobei der erste Regelsatz die Relevanz der Restinformationen für Diagnosezwecke auch auf der Basis von mindestens einem von: (a) bekannten medizinischen Zuständen des Patienten (103) oder (b) den erfassten medizinischen Daten definiert.

10. Medizinisches Diagnoseunterstützungsverfahren nach Anspruch 8, wobei die medizinischen Daten vom Körper des Patienten (103) erfasst und dem Arzt (124) während einer Online-Sitzung zwischen dem Patienten (103) und dem Arzt (124) angezeigt werden.

11. Medizinisches Diagnoseunterstützungsverfahren nach Anspruch 8, wobei die medizinischen Daten vom Körper des Patienten (103) erfasst und dem Arzt (124) zu einem dritten Zeitpunkt nach dem gegebenen Zeitpunkt angezeigt werden, wobei das medizinische Datenerfassungsgerät (104) nicht mit einem medizinischen Diagnoseunterstützungssystem (200) in Verbindung steht.

12. Medizinisches Diagnoseunterstützungsverfahren nach Anspruch 8, wobei die Restinformationen eines oder mehrere beinhalten von:
a. einem oder mehreren Luftverschmutzungsindikatoren;
b. einem oder mehreren Wasserverschmutzungsindikatoren;
c. Informationen über einen Krankheitsausbruch;
d. Informationen über eine Strahlungsbelastung;
e. Wetterinformationen;
f. Informationen über Lebensmittelvergiftungen; oder
g. bekannten Krankheiten am ersten Standort oder an den zweiten Standorten.

13. Medizinisches Diagnoseunterstützungsverfahren nach Anspruch 8, wobei die Restinformationen aus Online-Quellen erhalten werden, wobei mindestens eine der Online-Quellen außerhalb des medizinischen Diagnoseunterstützungssystems (200) liegt.

14. Medizinisches Diagnoseunterstützungsverfahren nach Anspruch 8, wobei das medizinische Datenerfassungsgerät (104) mindestens einen medizinischen Datenerfassungssensor (106) umfasst und wobei die medizinischen Daten mindestens einen vom medizinischen Datenerfassungssensor (106) erhaltenen Messwert umfassen.

15. Nicht-transitorisches, computerlesbares Speichermedium mit damit verkörpertem computerlesbarem Programmcode, wobei der computerlesbare Programmcode von mindestens einem Prozessor eines Computers ausgeführt werden kann, um ein Verfahren durchzuführen, das Folgendes umfasst:
Erhalten medizinischer Daten, die zu einem gegebenen Zeitpunkt mit Hilfe eines medizinischen Datenerfassungsgeräts (104) von einem Körper eines Patienten (103) erfasst werden;
Identifizieren und Abrufen von Restinformationen, die mit mindestens einem assoziiert sind von: (i) einem ersten Standort des Patienten (103) zu dem gegebenen Zeitpunkt oder (ii) an einem oder mehreren zweiten Standorten des Patienten (103) zu einem oder mehreren entsprechenden zweiten Zeitpunkten, die vor dem gegebenen Zeitpunkt liegen; wobei die abgerufenen Restinformationen anhand eines ersten Regelsatzes identifiziert werden, der eine Relevanz der Restinformationen für Diagnosezwecke definiert auf der Basis von mindestens einem von: (a) dem ersten Standort und dem gegebenen Zeitpunkt und Metadaten, die einen Zeitraum der Relevanz von Typen der Restinformationen definieren, oder (b) den zweiten Standorten, den entsprechenden zweiten Zeitpunkten und den Metadaten, die den Zeitraum der Relevanz von Typen der Restinformationen definieren; und
Anzeigen der medizinischen Daten und der Restinformationen einem Arzt (124), so dass der Arzt (124) eine Diagnose eines medizinischen Zustands des Patienten (103) stellen kann.

## Revendications

1. Système d'aide au diagnostic médical (200), le système d'aide au diagnostic médical (200) comprenant une ressource de traitement configurée pour :
obtenir des données médicales acquises à partir d'un corps d'un patient à un instant donné à l'aide d'un dispositif d'acquisition de données médicales (104) ;
identifier et récupérer des informations résiduelles associées à au moins l'un des éléments parmi : (i) un premier emplacement du patient (103) à l'instant donné, ou (ii) un ou plusieurs seconds emplacements du patient (103) à un ou plusieurs seconds instants correspondants antérieurs à l'instant donné ; dans lequel les informations résiduelles récupérées sont identifiées à l'aide d'un premier ensemble de règles définissant une pertinence des informations résiduelles à des fins de diagnostic sur la base d'au moins l'un des éléments parmi : (a) le premier emplacement et l'instant donné et des métadonnées définissant une durée de pertinence de types des informations résiduelles, ou (b) les seconds emplacements, les deuxièmes instants correspondants et les métadonnées définissant la durée de pertinence de types des informations résiduelles ; et
afficher les données médicales et les informations résiduelles à un praticien médical (124), permettant ainsi au praticien médical (124) de fournir un diagnostic d'une condition médicale du patient.

2. Système d'aide au diagnostic médical (200) selon la revendication 1, dans lequel le premier ensemble de règles définit la pertinence des informations résiduelles à des fins de diagnostic également sur la base d'au moins l'un des éléments parmi : (a) des conditions médicales connues du patient (103), ou (b) les données médicales acquises.

3. Système d'aide au diagnostic médical (200) selon la revendication 1, dans lequel les données médicales sont acquises à partir du corps du patient (103) et affichées au praticien médical (124) pendant une session en ligne entre le patient (103) et le praticien médical (124).

4. Système d'aide au diagnostic médical (200) selon la revendication 1, dans lequel les données médicales sont acquises à partir du corps du patient (103) et affichées au praticien médical (124) à un troisième instant, après l'instant donné, dans lequel le dispositif d'acquisition de données médicales (104) n'est pas en communication avec le système d'aide au diagnostic médical (200).

5. Système d'aide au diagnostic médical (200) selon la revendication 1, dans lequel les informations résiduelles incluent un ou plusieurs éléments parmi :
a. un ou plusieurs indicateurs de pollution de l'air ;
b. un ou plusieurs indicateurs de pollution de l'eau ;
c. des informations d'une flambée de maladie ;
d. des informations d'un niveau de radiation ;
e. des informations météorologiques ;
f. des informations d'intoxication alimentaire ; ou
g. des maladies connues au premier emplacement ou aux seconds emplacements.

6. Système d'aide au diagnostic médical (200) selon la revendication 1, dans lequel les informations résiduelles sont obtenues à partir de sources en ligne, dans lequel au moins l'une des sources en ligne est externe au système d'aide au diagnostic médical (200).

7. Système d'aide au diagnostic médical (200) selon la revendication 1, dans lequel le dispositif d'acquisition de données médicales (104) inclut au moins un capteur d'acquisition de données médicales (106), et dans lequel les données médicales incluent au moins une mesure obtenue par le capteur d'acquisition de données médicales (106).

8. Procédé d'aide au diagnostic médical, le procédé comprenant :
une obtention de données médicales acquises à partir d'un corps d'un patient (103) à un instant donné à l'aide d'un dispositif d'acquisition de données médicales (104) ;
une identification et une récupération d'informations résiduelles associées à au moins l'un des éléments parmi : (i) un premier emplacement du patient (103) à l'instant donné, ou (ii) un ou plusieurs seconds emplacements du patient (103) à un ou plusieurs deuxièmes instants correspondants antérieurs à l'instant donné ; dans lequel les informations résiduelles récupérées sont identifiées à l'aide d'un premier ensemble de règles définissant une pertinence des informations résiduelles à des fins de diagnostic sur la base d'au moins l'un des éléments parmi : (a) le premier emplacement et l'instant donné et des métadonnées définissant une durée de pertinence de types des informations résiduelles, ou (b) les seconds emplacements, les deuxièmes instants correspondants et les métadonnées définissant la durée de pertinence de types des informations résiduelles ; et
un affichage des données médicales et des informations résiduelles à un praticien médical (124), permettant ainsi au praticien médical (124) de fournir un diagnostic d'une condition médicale du patient (103).

9. Procédé d'aide au diagnostic médical selon la revendication 8, dans lequel le premier ensemble de règles définit la pertinence des informations résiduelles à des fins de diagnostic également sur la base d'au moins l'un des éléments parmi : (a) des conditions médicales connues du patient (103), ou (b) les données médicales acquises.

10. Procédé d'aide au diagnostic médical selon la revendication 8, dans lequel les données médicales sont acquises à partir du corps du patient (103) et affichées au praticien médical (124) pendant une session en ligne entre le patient (103) et le praticien médical (124).

11. Procédé d'aide au diagnostic médical selon la revendication 8, dans lequel les données médicales sont acquises à partir du corps du patient (103) et affichées au praticien médical (124) à un troisième instant, après l'instant donné, dans lequel le dispositif d'acquisition de données médicales (104) n'est pas en communication avec un système d'aide au diagnostic médical (200).

12. Procédé d'aide au diagnostic médical selon la revendication 8, dans lequel les informations résiduelles incluent un ou plusieurs éléments parmi :
a. un ou plusieurs indicateurs de pollution de l'air ;
b. un ou plusieurs indicateurs de pollution de l'eau ;
c. des informations d'une flambée de maladie ;
d. des informations d'un niveau de radiation ;
e. des informations météorologiques ;
f. des informations d'intoxication alimentaire ; ou
g. des maladies connues au premier emplacement ou aux seconds emplacements.

13. Procédé d'aide au diagnostic médical selon la revendication 8, dans lequel les informations résiduelles sont obtenues à partir de sources en ligne, dans lequel au moins l'une des sources en ligne est externe à un système d'aide au diagnostic médical (200).

14. Procédé d'aide au diagnostic médical selon la revendication 8, dans lequel le dispositif d'acquisition de données médicales (104) inclut au moins un capteur d'acquisition de données médicales (106), et dans lequel les données médicales incluent au moins une mesure obtenue par le capteur d'acquisition de données médicales (106).

15. Support de stockage lisible par ordinateur non transitoire ayant un code de programme lisible par ordinateur incorporé dans celui-ci, le code de programme lisible par ordinateur étant exécutable par au moins un processeur d'un ordinateur pour réaliser un procédé comprenant :
une obtention de données médicales acquises à partir d'un corps d'un patient (103) à un instant donné à l'aide d'un dispositif d'acquisition de données médicales (104) ;
une identification et une récupération d'informations résiduelles associées à au moins l'un des éléments parmi : (i) un premier emplacement du patient (103) à l'instant donné, ou (ii) un ou plusieurs seconds emplacements du patient (103) à un ou plusieurs deuxièmes instants correspondants antérieurs à l'instant donné ; dans lequel les informations résiduelles récupérées sont identifiées à l'aide d'un premier ensemble de règles définissant une pertinence des informations résiduelles à des fins de diagnostic sur la base d'au moins l'un des éléments parmi : (a) le premier emplacement et l'instant donné et des métadonnées définissant une durée de pertinence de types des informations résiduelles, ou (b) les seconds emplacements, les deuxièmes instants correspondants et les métadonnées définissant la durée de pertinence de types des informations résiduelles ; et
un affichage des données médicales et des informations résiduelles à un praticien médical (124), permettant ainsi au praticien médical (124) de fournir un diagnostic d'une condition médicale du patient (103).
